# EUROPEAN PATENT APPLICATION

(11) **EP 2 316 821 A1**
(43) Date of publication of application: **04.05.2011**
(21) Application number: 09174264.3
(22) Date of filing: 27.10.2009
(51) Int. Cl.: C07C 253/30, C07D 257/04

(54) **Process for the manufacture of organic compounds**

(71) Applicant: Novartis AG, 4056 Basel (CH)
(72) Inventor: The designation of the inventor has not yet been filed
(74) Representative: Roth, Peter Richard

(57) **Abstract**

The present invention relates to a process for the manufacture of an angiotensin receptor blocker (ARB: also called angiotension II receptor antagonist or AT₁ receptor antagonist) and salts, thereof, to novel intermediates and process steps.

## Description

### Field of the invention

The invention to to a novel process, novel process and novel intermediates in the synthesis of valsartan.

### Background of the invention

The present invention relates to a process for the preparation of valsartan. Valsartan, i.e (S)-*N*-(1-carboxy-2-methylprop-1-yl)-*N*-pentanoyl-*N*-[2'-(1*H*-tetrazol-5-yl)- biphenyl-4-ylmethyl]amine, an angiotensin ll receptor antagonists can be used e.g, for the of hypertension and has the following structure:

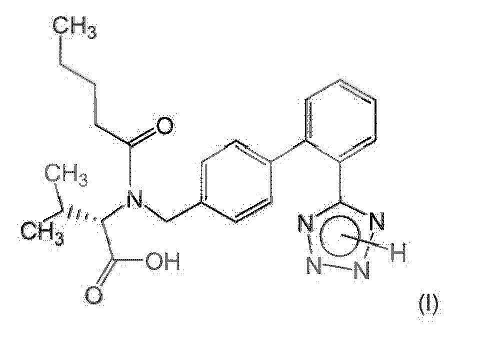

Valsartan and its synthesis are described in EP-A-0443983 and US ,in particular Examples 16, 37 and 54 thereof.

One of the key structural elements of valsartan is its tetrazole moiety. Various methods of preparing tetrazoles are in the literature. For example, it is known in the art that tetrazole derivatives can be prepared by reacting a cyano group with an azide reagent, that is a process which involves a [3+2] cycloaddition reaction leading to the formation of 5-substituted tetrazoles. For example, EP 0536400 the preparation of a tetrazole compound by reacting a cyano group with hydrazoic acid or a salt thereof, such as metal salts, for example, alkali or earth alkali metal (eg. sodium azide, potassium azide, calcium azide, magnesium azide, aluminium azide or tin azide) and salts with organic bases (eg. tetramethylguanidinium azide). It is also known that tetrazoles can be prepared, for example as described in US 4,874,867, by reacting a cyano group with a tin azide, Tetrazole forming methods which use tin azides need special care in production processes, because of ecological problems, and require a significant amount of additional process steps to recycle them the wastewater, thereby additionally increasing the production costs. An environmentally friendly alternative to the use of tin azides is the use trialkylammonium azides or tetraalkylammonium azides, however when using such reagents volatile sublimates may form in the reaction reactors, which have the risk of explosion and are not easy to handle in large scale production.

There is a strong need to develop new tetrazole-forming process variants, that the above-mentioned disadvantages. Organoboron azides and organoaluminium azides have been to offer an attractive to tin azides in [2+3]cycloadditions with nitrites to form tetrazoles. Said boron and aluminium compounds, in particular said aluminium compounds, are available in considerably large and are inexpensive. In particular, as described in WO 2005/014602, these azides are useful in a process for producing valsartan comprising reacting a compound of formula (ll), or an ester thereof, 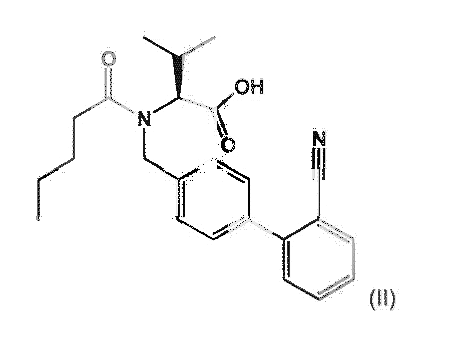 wherein a preferred ester of a compound of formula (11) is, for example a benzyl ester thereof, the formula (111-a)

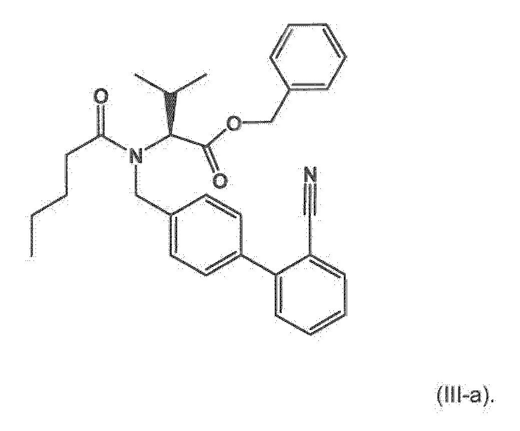

Thus, in one embodiment, the tetrazole-forming methodology described in WO 2005/014602 relates to a one-pot process wherein by the use of a single azide reagent two reaction steps take place: the conversion of an ester moiety into an acid moiety and the conversion of a cyano group into a tetrazole. A one-pot process the happening of two or more reaction steps without isolation of the intermediate species, Therefore, they are industrially attractive synthetic methods because they reduce work-upsteps, thus reducing the quantity of solvents required and the time and labor required between consecutive manufacture process steps.

In the above-mentioned one-pot process, it is observed that the conversion of the ester moiety into the acid moiety leads to acyl-azide by-products, which in turn may form isocyanate and/or carbamate by-products. The conversion of an alkyl ester moiety into an acyl-azide in the presence of an azide reagent is also known to the skilled person from literature references such as Tetrahedron Letters, 1994, 35, 4947. The formation of acyl-azide by-products is undesirable because are formed at the expense of a decrease yield of the desired acid product. Thus, the object of the present invention is to provide an alternative process that has many of the advantages of the process described in WO 2005/014602 but that avoids the formation of said by-products.

It is found that the present invention meets the objective and thus provides a method to convert an ester of a compound of formula (ll), preferably an ester as described below, into valsartan. Accordingly, the process according to the present invention shows one or more of the following advantages: (1) it does not require a process step wherein a tin azide is used and therefore it is environmentally friendly; (2) it is economically attractive; (3) it may be carried out on a large scale; (4) it may be carried out in a one-pot fashion thus reducing the time and labor required for procedures such as isolation of intermediate products and solvent replacement; (5) it affords enantiomerically pure target products; and (6) it avoids the formation of the above-mentioned side products. Thus, the preparation methods of the present invention are advantageous for the industrial preparation of valsartan,

### Summary of the Invention

As explained above, an embodiment of the invention described in WO 2005/014602 relates to a one-pot process wherein two chemical steps, namely conversion an ester into an acid and a tetrazole formation step, take place without the isolation of intermediate species to provide valsartan.

During an investigation into an alternative one-pot process to prepare valsartan, a novel method for converting the ester group of a compound of formula (ll), as defined herein, into the free acid of formula (ll), as described above, was found, Namely, in one aspect, the present invention relates to the conversion of an ester group, such as a benzyl ester, into a free acid by the use of an organoaluminium halide reagent. The use of an organoaluminium halide reagent to effect such a chemical reaction provides many advantages, as explained hereinafter. Typically a benzyl ester is converted into a free acid under hydrogenation conditions (i.e, with hydrogen in the presence of a transition metal catalyst, such a palladium catalyst). By using an organoaluminium halide reagent, the use of flammable hydrogen is avoided, no pressurized reactors are necessary and expensive transition metal catalysts are not needed. Morover, the use of an organoaluminium halide reagent is advantageous both in terms of toxicity and costs.

In Tetrahedron Letters, 1979, 2793, it is reported that A1C1₃ can effect the removal of a benzyl ester group to yield the free acid. Therefore, the finding that an organoaluminium halide can effect the conversion of an ester into a free acid is new and fully surprising. In addition, the use of an organoaluminium halide reagent provides means to prepare in-situ an organoaluminium azide reagent, and thus allows the subsequent conversion of a cyano group into a tetrazole group, without having to isolate the free add intermediate. Therefore, in a further embodiment, the present invention also allows the preparation of valsartan via a one-pot process, wherein the use of an organoaluminium halide reagent effects the conversion of an ester group into an acid group and the use of an azide reagent, such as an organoaluminium azide reagent, preferably prepared in-situ from the organoaluminium halide reagent, effects the conversion of a cyano group into a tetrazole group.

### Detailed Description of the invention

Process for preparing compounds of formula (lll), (lV) and (V), as defined herein below, have been described in the literature. For example, the preparation of a compound of formula (lV) has been described in the Chinese Journal of Pharmaceuticals 2001, 32(9), 385 and in EP1533305A1. By using standard methods, known to the person skilled in the art, for example as described in Richard C. Larock, "Comprehensive Organic Transformations: A Guide to Functional Group Preparations", Second Edition, Wiley-VCH Verlag GmbH, 2000, the compound of formula (lll) can be converted into a compound of formula (lV) via a esterification reaction. In particular, the preparation of a compound of formula (lV), wherein R is benzyl, has been described in Organic Process Research & Development 2007, 11,892.

### B. Conversion of an ester group into a free acid with an organoaluminium halide reagent

### B. 1: Conversion of an ester group into a free acid in a cyano-containing compound

In a first aspect, the invention relates to a process for the manufacture of a compound of formula (lV) or salt thereof,
such as an amine salt thereof, 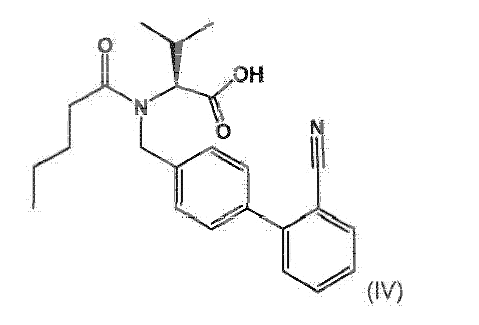 comprising
reacting a compound of formula (lll), or salt thereof, 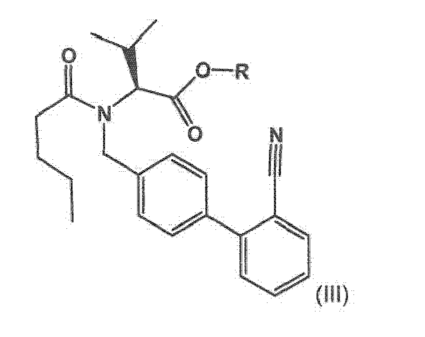 wherein R is C₁₋₇alkyl, 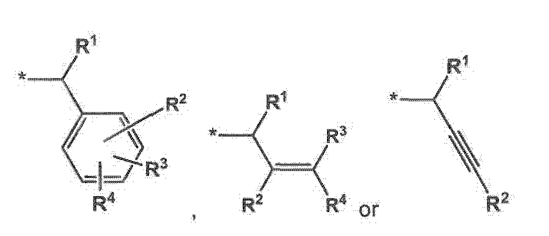 wherein R1 is hydrogen, C₁₋₇alkyl, C₁₋₇alkoxy, C₃₋₈ cycloalkyl or C₆₋₁₀aryl, each unsubstituted or substituted by one or more, e.g, up to three, substituents selected from , halo, nitro, C₁₋₇alkyl, halo -C₁₋₇alky, C₁₋₇alkoxy-C₁₋₇alkyl, C₁₋₇alkoxy, halo₋C₁₋₇alkoxy, C₁₋₇alkoxy-C₁₋₇alkoxy, halo-C₁₋₇alkoxy-C₁₋₇alkoxy, C₁₋₇alkyₗ-C₁₋₇alkoxy, halo-C₁₋₇alkyl-C₁₋₇alkoxy; and
R2, R3 and R4 are the same or different form each other are hydrogen, halo, nitro, C₁₋₇alkyl, C₁₋₇alkoxy, C₃₋₈cycloalkyl or C₆₋₁₀aryl, each unsubstituted or substituted by one or more, e.g. up to three, substituents selected from , halo, nitro, C₁₋₇alkyl, halo- C₁₋₇alkyl, C₁₋₇alkoxy-C₁₋₇alkyl, C₁₋₇alkoxy, hato-C₁₋₇alkoxy, C₁₋₇alkoxy-C₁-₇alkoxy, halo-C₁₋₇alkoxy-C₁₋₇alkoxy, C₁₋₇alkoxy, C₁₋₇alkyl, halo-C₁₋₇alkyl-C₁-₇alkoxy;
with an organoaluminiun halide of formula R5R6AlX or R5AlX₂
wherein R5 and R6 are, independently from one another, C₁₋₇alkyl, C₃₋C₈alkenyl, C₃₋₈cycloalkyl or C₆₋₁₀aryl, preferably C₁₋₇alkyl, and X is halogen,
to obtain the compound of formula (lV).

In particular, a process for the manufacture of a compound of formula (lV) or salt thereof,
such as an amine salt thereof, 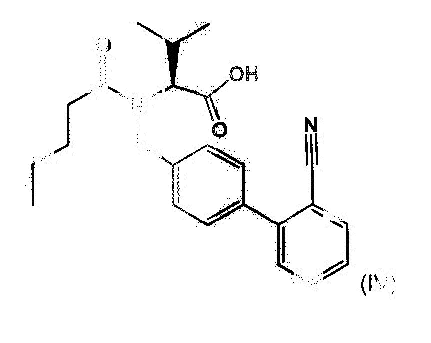 comprising
reaching compound of formula (lll), or salt thereof, 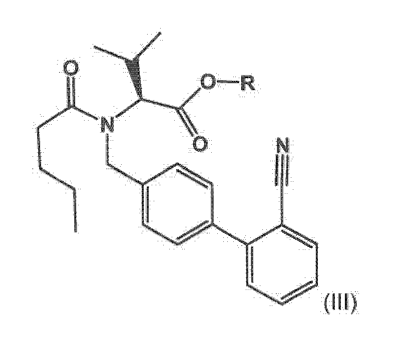 wherein R is C₁₋₇alkyl, 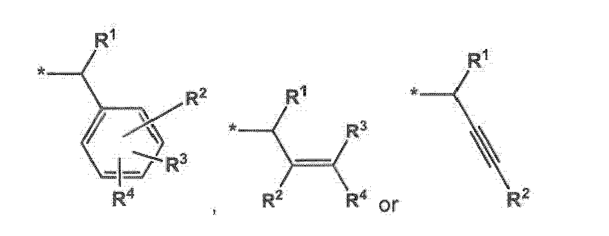 wherein R1 is hydrogen, C₁₋₇alkyl, C₁₋₇alkoxy, C₃₋₈cycloalkyl or C₆₋ₗ₀aryl, preferably hydrogen; and
R2, R3 and R4 are the same or different form each other and are hydrogen, halo, nitro, C₁₋₇alkyl, C₁₋₇alkoxy, C₃₋₈cycloalkyl or C₆₋ₗ₀aryl, preferably hydrogen,
with an organoaluminiun halide of formula R5AIX₂
wherein R5 and R6 are, independently from one another, C₁₋₇alkyl, C₃₋C₈alkenyl, C₃. ₈cycloalkl, preferably C₁₋₇alkyl, and X is halogen,
to obtain the compound of formula (lV).

Preferably, a process for the manufacture of a compound of formula (lV) or salt thereof,
such as an amine salt thereof, 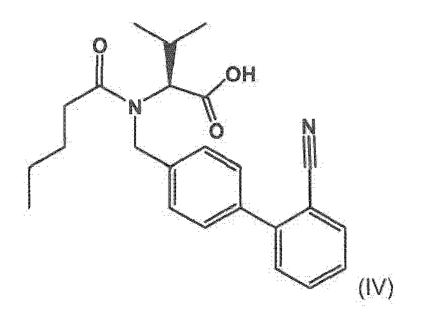 comprising
reaching a compound of formula (lll), or salt thereof, 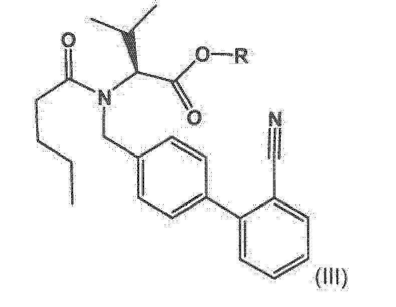 wherein R is 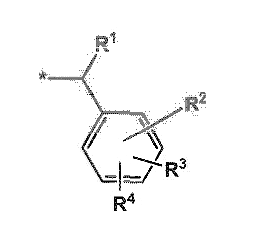 wherein R1 is hydrogen, C₁₋₇alkyl, C₁-₇alkoxy, C₃₋₈cycloalkyl or C₆₋₁₀aryl, preferably hydrogen; and
R2, R3 and R4 are the same or different form each other and are hydrogen, halo, nitro, C₁₋₇alkyl, C₁-₇alkoxy, C₃₋₈cycloalkyl or C₆₋₁₀aryl, preferably hydrogen or C₁₋₇alkoxy, most preferably hydrogen,
with an organoaluminiun halide of formula R5R6A1X or R5A1X₂
wherein R5 R6 independently from one another, C₁-₇alkyl, C₃-C₈alkenyl ₈cycloalkyl or C₆₋₁₀aryl, preferably C₁₋₇alkyl , and X is halogen,
to obtain the compound of (lV).

Preferably, the R, for any of the processes above its benzyl, p-methoxybenzyl, allyl, cinnamyl, prenyl or propargyl, more preferably, benzyl.

In any of the processes above detailed, preferably, the organoaluminiun halide is of formula R5R6AlX, wherein R5, R6 and X are as defined above, and is for example diethylaluminium chloride or dimethylaluminium chloride, most preferably diethylaluminium chloride.

The molar ratio of an organoaluminiun halide, as described herein, to a compound of formula (lll), as described herein, is for example 6:1, such as 5:1, preferably 4:1or 3:1_{.}

ln any of the processes above detailed, the reaction temperature is preferably in the temperature range of from room temperature to the boiling point of the solvent, for example, reaction temperature range is of from 20°C to 170°C, preferably, of from 60°C to 130°C.

### B.2: Conversion of an ester group into a free acid in a tetrazole-containing compound: synthesis of valsartan

In a further aspect, the invention relates to a process for the manufacture of a compound of formula (l), or salt thereof, 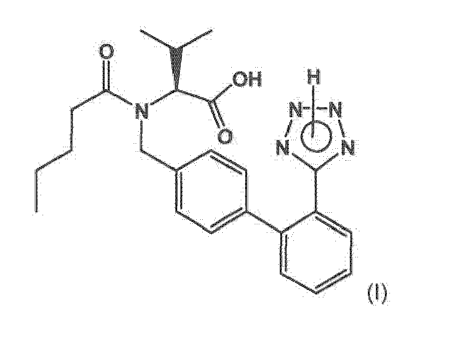 comprising
reacting a compound of formula (V), or salt thereof, 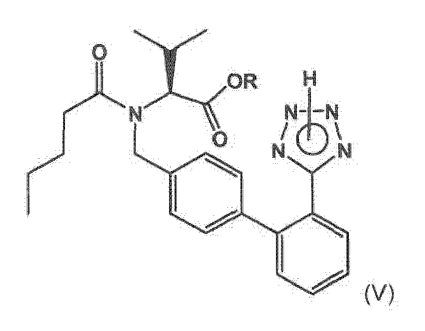 wherein R is C₁-₇alkyl, 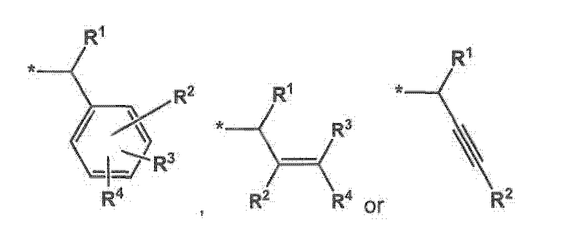 wherein R1 is hydrogen, C₁₋₇alkyl, C₃-₈cycloalkyl or C₆₋₁₀aryl, each unsubstituted or substituted by one or more, e.g. up to three, substituents selected from _{,} halo, nitro, C₁₋₇alkyl, halo- C₁₋₇alkyl, C₁₋₇alkoxy-C₁₋₇alkyl, C₁-₇alkoxy, halo-C₁₋₇alkoxy, C₁₋₇alkoxy-C₁₋₇alkoxy, halo-C₁₋₇alkoxy-C₁₋₇alkoxy, C₁-₇alkyl-C₁₋₇alkoxy, halo-C₁-₇alkyl-C₁₋₇alkoxy; and
R2, R3 and R4 are the same or different form each other are nitro, C₁-₇alkyl, C₁-₇alkoxy, C₃-₈₀cycoalkyl or C₆₋₁₀aryl, each unsubstituted or substituted by one or more, e,g. up to three, substituents selected from , halo, nitro, C₁₋₇alkyl, halo- C₁₋₇alkyl, ₇alkoxy-C₁₋₇alkyl, C₁-₇alky, halo-C₁-₇alkoxy, C₁-₇alkoxy-C₁₋₇alkoxy, halo-C₁₋₇alkoxy-C₁₋₇alkoxy, C₁₋₇alkyl-C₁₋₇alkoxy, halo-C₁₋₇alkyl-C₁₋₇alkoxy;
with an organoaluminiun halide of formula R5AlX₂
wherein R5 and R6 are, independently from one another, C₁-₇alkyl, C₃-C₈alkenyl, C₃₋₈cycloalkyl or C₆₋₁₀aryl, preferably C₁-₇alkyl, and X is halogen,
to obtain the compound of formula (l).

In particular, a process for the manufacture of a compound of formula (l) or salt thereof, 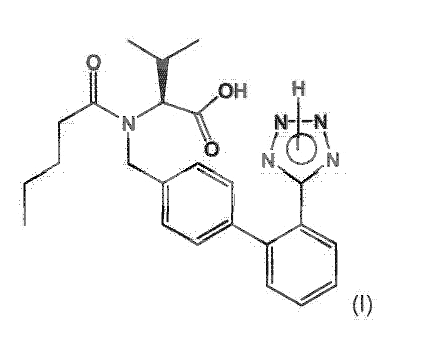 comprising
reacting a compound of (V), or salt thereof, 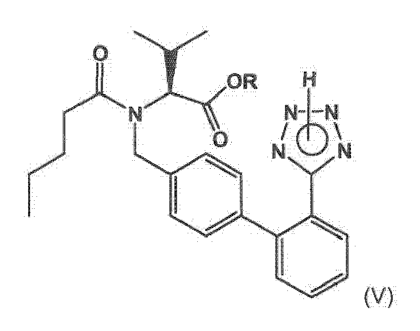 wherein R is C₁₋₇alkyl, 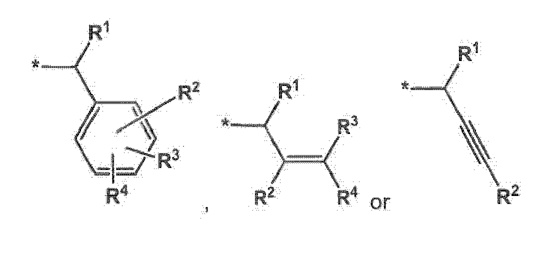 R1 is hydrogen, C₁₋₇alkyl, C₁₋₇alkoxy, C₃₋₈cycloalkyl or C₆.₁₀aryl, preferably hydrogen; and
R2, R3 and R4 are the same or different form each other and are hydrogen, halo, nitro, C₁-₇alky), C₁₋₇alkoxy, C₃-₈cycloalkyl or C₆₋₁₀aryl, preferably hydrogen,
with an organoaluminiun halide of formula R5R6AlX or R5AlX₂
wherein R5 R6 are, independently from one another, C₁₋₇alkyl, C₃. ₈cycloalkyl or C₆₋₁₀aryl, preferably C₁.₇alkyl, and X is halogen,
to obtain the compound of formula (l).

Preferably, a process for the manufacture of a compound of formula (l) or salt thereof, 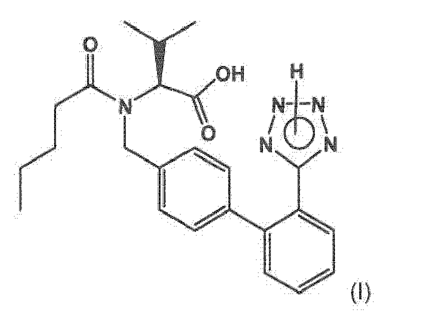 comprising
reaching a compound of formula (V), or salt thereof, 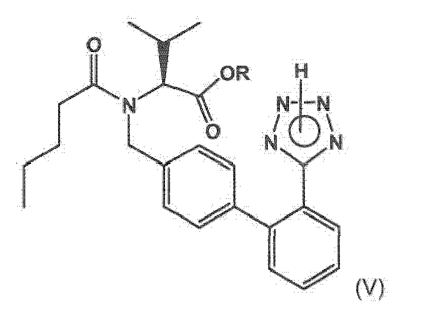 wherein R is 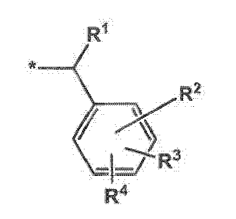 wherein R1 is hydrogen, C₁₋₇alkyl, C₁₋₇alkoxy,or C₆₋₁₀aryl, preferably hydrogen; and
R2, R3 and R4 are the same or different form each other and are hydrogen, halo, nitro, C₁₋₇alkyl, C₁-₇alkoxy, C₃₋₈cycloalkyl or C₆₋₁₀aryl, preferably hydrogen or C₁₋₇alkoxy, most preferably hydrogen,
with an organoaluminiun halide of formula R5R6AlX or R5AlX₂
wherein R5 and R6 are, independently from one another, C₁₋₇alkyl, C₃₋C₈alkenyl,C₃. ₈cycloalkyl or C₆₋₁₀aryl, preferably C₁₋₇alkyl, and X is halogen,
to obtain the compound of formula (lV).

Preferably, the group R, for any of the processes above detailed, is benzyl, p-methoxybenzyl, allyl, cinnamyl, prenyl or propargyl, more preferably, benzyl,

In any of the processes above detailed, the organoaluminiun halide is of formula R5R6AlX, wherein R5, R6 and X are as defined above, and is for example diethylaluminium chloride or dimethylaluminium chloride, most preferably diethylaluminium chloride.

The molar ratio of an organoaluminiun halide, as described herein, to a of formula (V), as described herein, is for example 6:1, such as 5:1, preferably 4:1 or 3:1.

In any of the processes above detailed, the reaction temperature is preferably in the temperature range of from room temperature to the boiling point of the for example, a reaction temperature range is of from 20°C to 170°C^{,} preferably, of from 60°C to 130°C_{.}

### C. Synthesis of valsatan via conversion of an ester group into a free acid with an organoaluminium halide reagent followed by conversion of a cyano group into a tertrazole.

### C.1: Step-wise synthesis of valsartan

This section relates to a process for the manufacture of valsartan, wherein the conversion of an ester group into a free acid and the conversion of a cyano group into a tetrazole take place step-wise, i.e. in two separate steps with isolation of the intermediate species.

In another aspect, the invention relates to a step-wise process for the manufacture of a compound of formula (l), or salt thereof, 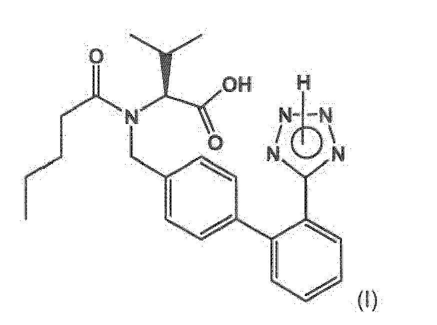 comprising the steps of
i) reacting a compound of formula (lll), or salt thereof, 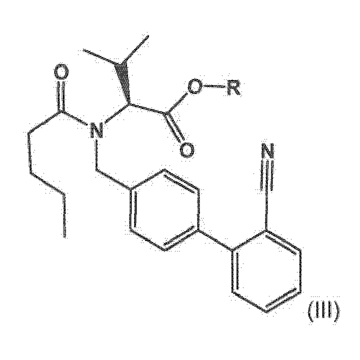 wherein R is C₁-₇alkyl, 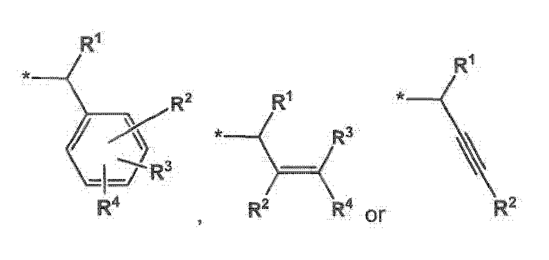 wherein R1 is hydrogen, C₁-₇alkyl, C₁-₇alkoxy, C₃₋₈cydoalkyl or C₆₋₁₀aryl, each unsubstituted or substituted by one or more, e.g. up to three, substituents selected from, halo, nitro, C₁₋₇alkyl, halo- C_{1-7alkyl}, C₁₋₇alkoxy-C₁-₇alkyl, C₁₋₇alkoxy, halo-C₁₋₇alkoxy, C₁₋₇alkoxy-C₁₋₇alkoxy, halo-C₁₋₇alkoxy,-C₁₋₇alkoxy C₁₋₇alky)-C₁₋₇alkoxy, halo-C₁₋₇alkyl-C₁₋₇alkoxy; and
   R2, R3 and R4 are the same or different form each other and are hydrogen, halo, nitro, C₁₋₇alkyl, C₁-₇alkoxy, C₃-₈cydoalkyl or C₆₋₁₀aryl, each unsubstituted or substituted by one or more, e.g. up to three, substituents selected from , halo, nitro, C₁₋₇alkyl, halo- C₁. ₇alkyl, C₁₋₇alkoxy-C1-7alkyl, C₁₋₇alkoxy, halo-C₁₋₇alkoxy, C₁₋₇alkoxy-C₁₋₇alkoxy, halo-C₁₋₇alkoxy-C₁₋₇alkoxy, C₁₋₇alkyl-C₁₋₇alkoxy, halo-C₁₋₇alkyl-C₁₋₇alkoxy;
   with an organoaluminiun halide of formula R5R6AlX or R5AlX₂
   wherein R5 and R6 are, independently from one another, C₁₋₇alkyl, C₃-C₈alkenyl,C₃. ₈cycloalkyl or C₆₋₁₀aryl, preferably C₁₋₇alkyl, and X is halogen,
   to obtain the compound of formula (lV), or salt thereof,
   such as an amine salt thereof, 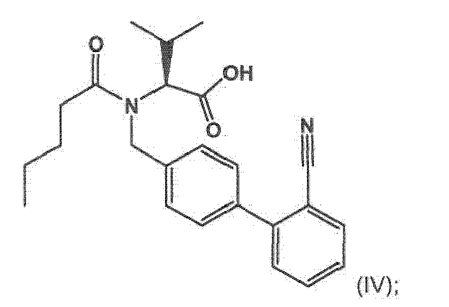
ii) treating the compound of formula (lV), or salt thereof,
   such as an amine salt thereof,
   with an azide reagent to provide compound of formula (l).

In another the invention relates to a process for the manufacture of a compound of formula (l), or salt thereof, 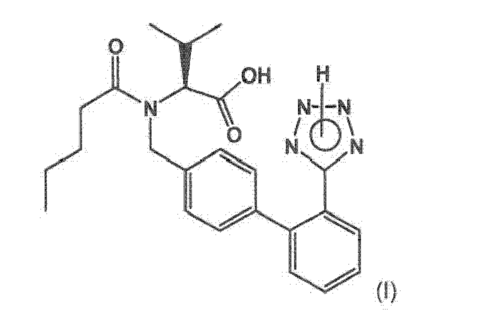 comprising
i) reacting a compound of formula (lll), or salt thereof, 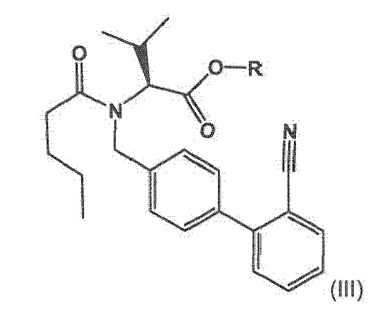 wherein R iS C₁-₇alkyl, 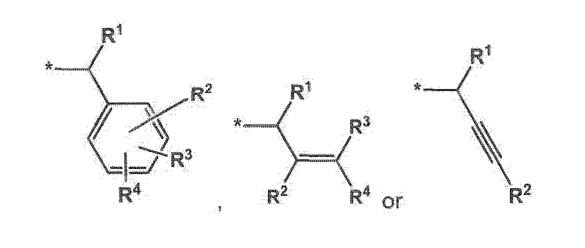 wherein R1 is hydrogen, C₁₋₇alkyl, C₁₋₇alkoxy, C₃₋₈cycloalkyl or C₆₋₁₀aryl, preferably hydrogen; and
   R2, R3 and R4 are the same or different form each other and are hydrogen, halo, nitro, C₁₋₇alkyl, C₁₋₇alkoxy, C₃₋₈cycloalkyl or C₆₋₁₀aryl, preferably hydrogen,
   with an organoaluminiun halide of formula R5R6AlX or R5AlX₂
   wherein R5 and R6 are, independently from one another, C₁-₇alkyl, C₃-C₈alkenyl, C₃₋₈cycloalkyl or C₆₋₁₀aryl, preferably C₁-₇alkyl, and X is halogen,
   to obtain the compound of formula (lV), or salt thereof,
   such as an amine salt thereof, 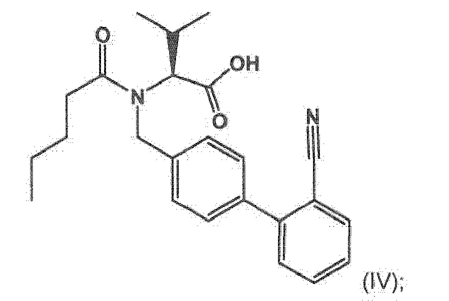
ii) treating the compound of formula (lV), or salt thereof,
   such as an amine salt thereof,
   with an azide reagent to provide compound of formula (l).

In another aspect, the invention relates to a process for the manufacture of a compound of formula (l), or salt thereof, 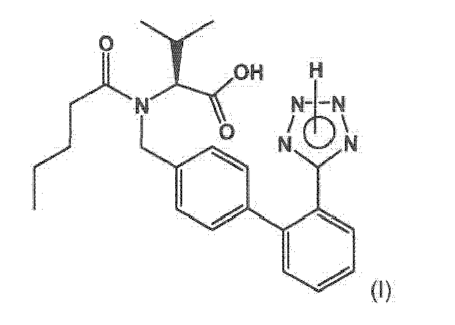 comprising
i) reacting a compound of formula (lll), or salt thereof, 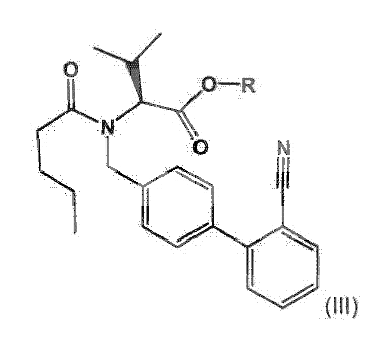 whereon R 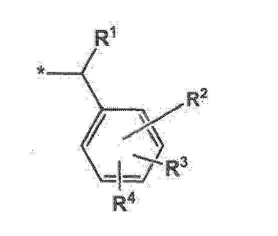 wherein R1 is hydrogen, C₁₋₇alkyl, C₁-₇alkoxy, C₃₋₈cycloalkyl or C₆₋₁₀aryl, preferably hydrogen, and
   R2, R3 and R4 are the same or different form each other and are hydrogen, nitro, C₁₋₇alkyl, C₁₋₇alkoxy, C₃₋₈cycloalkyl orC₆₋₁₀aryl, preferably hydrogen or C₁₋₇alkoxy, more preferably hydrogen,
   with an organoaluminiun halide of formula R5R6AlX or R5AlX₂
   wherein R5 and R6 are, independently from one another, C₁-₇alkyl, C₃-C₈alkenyl, C₃₋₈cycloalkyl or C₆₋₁₀aryl, preferablyC1-7alkyl, and X is halogen,
   to obtain the compound of formula (lV), or salt thereof,
   such as an amine salt thereof, 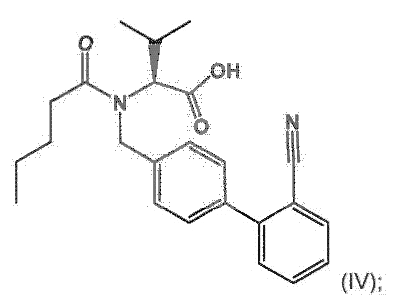
ii) treating the compound of (lV), or salt thereof,
   such as an amine salt thereof,
   with an azide reagent to provide compound of formula (l).

Preferred azide reagents are, for example, metal salts of hydrazoic azid, such as alkali or earth alkali metal salts, (eg. lithium azide, sodium azide, potassium azide, calcium azide, magnesium azide, aluminium azide), salts of hydrazoic acid with organic bases (eg. tetramethylguanidinium azide), or an azide of formula R7R8MN₃ wherein M is boron or aluminium, preferably aluminium, R7 and R8 are, independently from one C₁₋₇alkyl, C₃-C₈alkenyl, C₃-₈cycloalkyl, C₃₋₈cycloalkyl-C₁-₇alkyl or C₆₋₁₀aryl-C₁-₇alkyl, preferably C₁₋₇alkyl, In particular, the azide reagent is of formula R7R8MN₃ wherein M is aluminium or boron, preferably aluminium, R7 and R8 are, independently from one another, C₁₋₇alkyl, C₃₋C₈alkenyl, C₃₋₈cyrloalkyl, C₃₋₈cycloalkyl-C₁₋₇alkyl or C₆₋₁₀aryl-C₁₋₇alkyl, preferably C1-7alkyl, Preferably, the azide reagent is diethylaluminium azide or dimethylaluminium azide, more preferably diethylaluminium azide.

Preferably, the group R, in any of the processes above detailed, is benzyl, p-methoxybenzyl, allyl, cinnamyl, prenyl or propargyl, more preferably, benzyl.

In any of the processes above detailed, preferably, the organoaluminiun halide is of formula R5R6AlX, wherein R5, R6 and X are as defined above, and is for example diethylaluminium chloride or dimethylatuminium chloride, most preferably diethylaluminium chloride.

The molar ratio of an organoaluminiun halide, as described herein, to a compound of formula (lll), as described herein, is for example 6:1, such as 5:1, preferably 4:1 3:1.. In any of the processes above detailed, the reaction temperature of i) is preferably in the temperature range of from room temperature to the boiling point of the solvent, for example, a reaction temperature range is of from 20°C to 170°C, preferably, of from 60°C to 130°C.

In any of the processes above detailed, the reaction temperature of step ii) is preferably in the temperature range of from room temperature to the boiling point of the solvent, for example, a reaction temperature range is of from 20°C to 170°C, preferably, of from 60°C to 130°C.

### C.2: One-pot synthesis of valsartan

This section relates to a process for the manufacture of valsartan, wherein the conversion of an ester group into a free acid and the conversion of a cyano group into a tetrazole take place in a one-pot process,

In another aspect, the invention relates to a one-pot process for the manufacture of a compound of formula (l), or salt thereof, 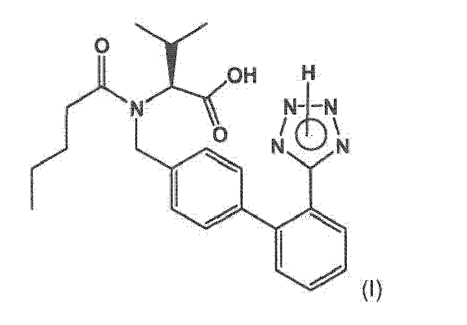 comprising
i) reacting a compound of formula (lll), or salt thereof, 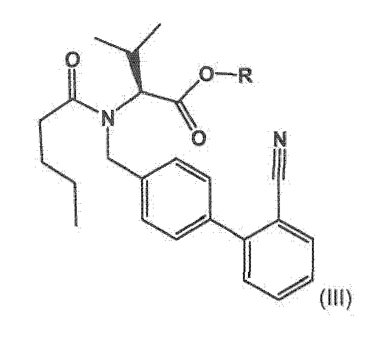 wherein R is C₁₋₇alkyl, 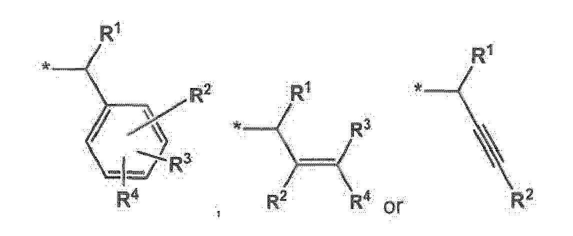 wherein R1 is hydrogen, C₁₋₇alkyl, C₁₋₇alkoxy, C₃₋₈cyloalkyl or C₆₋₁₀aryl, each unsubstituted or substituted by one or more, e.g up to three, substituents selected from , halo, nitro, C₁₋₇alkyl, halo- C₁₋₇alkyl, C₁₋₇alkoxy-C₁₋₇alkyl, Cₗ₋₇alkoxy, halo-C₁₋₇alkoxy, C₁₋₇alkoxy-C₁₋₇alkoxy, halo-C₁₋₇alkoxy-C₁₋₇alkoxy**,** C₁₋₇alkyl-C₁₋₇alkoxy, halo-C₁₋₇alkyl-C₁. ₇alkoxy; and
   R2, R3 and R4 are the same or different form each other and are hydrogen, halo, nitro, C₁₋₇alkyl, C₃₋₈cydoalkyl or C₆₋₁₀aryl, each unsubstituted or substituted by one or more, e.g. up to three, substituents selected from , halo, nitro, C₁₋₇alkyl, halo- C₁₋₇alkyl, C₁₋₇alkoxy-C₁₋₇alkyl, C₁₋₇alkoxy, halo-C₁₋₇alkoxy, C₁₋₇alkoxy-C₁₋₇alkoxy, halo-C₁₋₇alkoxy-C₁₋₇alkoxy, halo-C₁₋₇alkyl-C₁₋₇alkoxy;
   with an organoaluminiun halide of formula R5R6AIX or R5AIX₂
   wherein R5 and R6 are, independently from one another, C₁₋₇alkyl, Cg. ₈cycloalkyl or C₆₋₁₀aryl, preferably C₁₋₇alkyl, and X is halogen,
   to obtain the compound of formula (IV) , or salt thereof,
   such as an amine salt thereof, 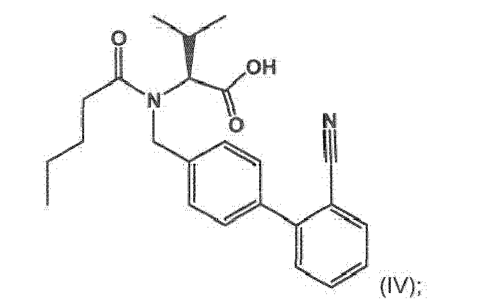
ii) adding to the resulting reaction mixture of step i) an azide reagent to obtain the compound of formula (I).

In another aspect, the invention relates to a one-pot process for the manufacture of a compound of formula (I), or salt thereof, 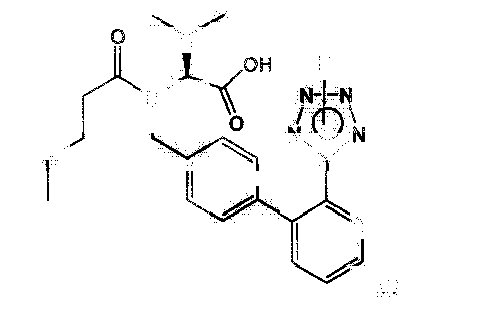 comprising
i) reacting a compound of formula (III), or salt thereof, 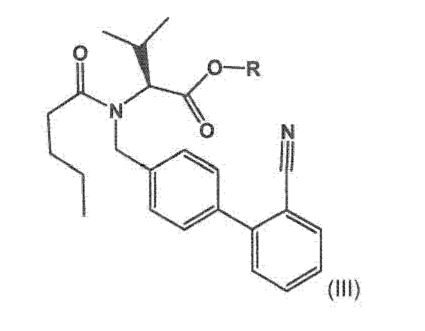 wherein R is C₁₋₇alkyl, 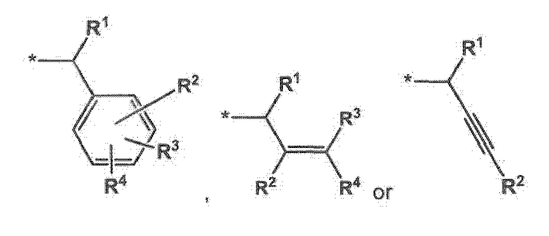 wherein R1 is hydrogen, C₁₋₇alkyl, C₁₋₇alkoxy, C₃₋₈cycloalkyl or C₆₋₁₀aryl, preferably hydrogen; and
   R2, R3 and R4 are the same or different form each other and are hydrogen, halo, nitro, C₁₋₇alkyl, C₃₋₈cydoalkyl or C₆₋₁₀aryl, preferably hydrogen,
   with an organoaluminiun halide of formula R5R6AIX or or R5AIX₂
   wherein R5 and R6 are, independently from one another, C₁₋₇alkyl, C₃-C₈alkenyl, C₃. ₈cycloalkyl or C₆₋₁₀aryl, preferably C₁₋₇alkyl, and X is halogen,
   to the compound of formula (IV), or salt thereof,
   such as an amine salt thereof, 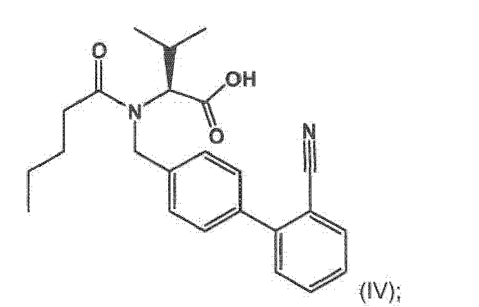
ii) adding to the resulting reaction mixture of step i) an azide reagent to obtain the compound of formula (I).

Preferred azide reagents are, for example, metal of of hydrazoic azid, as alkali or earth alkali metal salts, (eg, lithium azide, sodium azide, potassium azide, calcium azide, magnesium azide, aluminium azide), salts of hydrazoic acid with organic bases (eg. tetramethylguanidinium azide), or an azide of formula R7R8MN₃ wherein M is boron or aluminium, preferably aluminium, R7 and R8 are, independently from one another, C₁₋₇alkyl, C₃-C₈alkenyl, C₃₋₈cydoalkyl, or C₆₋₁₀aryl-C₁₋₇alkyl, preferably C₁₋₇alkyl. In the azide reagent is of formula R7R8MN3 wherein M is aluminium or boron, preferably R7 and R8 are, independently one another, C₁₋₇alkyl, C₃-C₈alkenyl, C₃₋₈cycloalkyl, C₃₋₈cyclalky-C₁₋₇alkyl or C₆₋₁₀aryl-C₁. ₇alkyl, preferably C₁₋₇alkyl, preferably, the azide reagent is diethylaluminium azide or dimethylaluminium azide, more preferably diethylaluminium azide.

In a preferred embodiment, the azide reagent is of formula R7R8MN₃, wherein M is aluminium, R7 and R8 are as defined and is prepared by adding a metal salt of hydrazoic for example alkali or earth alkali metal salts, (eg. lithium azide, sodium azide, potassium azide, calcium azide, magnesium or aluminium azide) to an organoaluminium halide of formula R5R6MX, as defined herein, Preferably, said in-situ preparation takes place by the metal salt of hydrazoic to the reaction mixture of step i). In this preferred the conversion of the organoaluminium halide to the organoaluminiumazide reagent takes art room temperature the tetrazole formation reaction takes place with heating above room temperature, preferably, of from 60'0 to 130°C, such as of from 90°C to 130°C. In this embodiment, an excess amount, for example 2 or more equivalents, of the azide reagent be used,

Preferably, the group R, in any of the processes above detailed, is benzyl, p-methoxybenzyl, allyl, cinnamyl, prenyl or propargyl, more preferably, benzyl.

In of the processes above detailed, preferably, the organoaluminiun halide is of formula R5R6AIX, wherein R5, R6 and X are as defined above, and is for example diethylaluminium chloride or dimethyl aluminium chloride, most preferably diethylaluminium chloride.

The molar of an organoaluminiun halide, as described herein, to a compound of formula (lll), as described herein, is for example 6:1, such as 5:1, preferably 4:1 or 3:1.

In of the processes above detailed, the reaction temperature of step i) is preferably in the temperature range of from room temperature to the boiling point of the solvent, for example, a reaction temperature range is of from 20°C to 170°C, preferably, of from 60°Cto13<TC.

In any of the processes above detailed, the reaction temperature of step ii) is preferably in the temperature range of from room temperature to the boiling point of the solvent, for example, a reaction temperature range is of from 20°C to 170°C, preferably, of from 60°C to 130°C, preferably of from 80°C to 130°C.

### D. Further objects of the invention

In the processes shown above several novel and inventive are involved. Consequently, further subjects of the present are the compounds shown below.

An amine salt of the compound of formula (IV), as defined above.

A compound of formula (lll), or salt thereof, 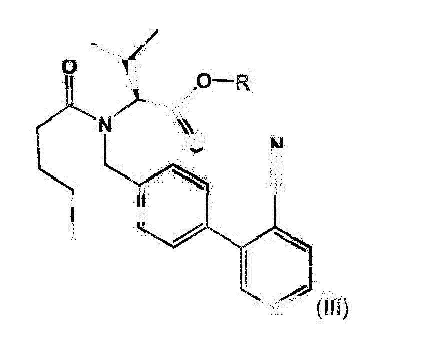 wherein R is is benzyl, allyl, cinnamyl, prenyl or propargyl, more preferably, benzyl.

Listed below are definitions of various terms used to describe the novel intermediates and synthesis steps of the present invention. These definitions, by replacing one, more than or all general expressions or symbols used in the present disclosure and thus yielding embodiments of the invention, in particular apply to the terms as they are used throughout the specification unless they are otherwise limited in specific instances either individually or as part of a larger group.

The term "C₁₋C₂₀₋" defines a moiety with up to and including maximally 20, especially up to and maximally 7, carbon atoms, said moiety being branched (one or more times) or straight-chained and bound via a terminal or a non-terminal carbon,

The term alkyl, as a radical or part of a radical, defines a moiety with up to and including maximally 7, C₁₋₇alkyl, in particular up to and including maximally 4, C₁₋₄alkyl, carbon atoms, said moiety being branched (one or more times) or straight-chained and bound via a terminal or a non-terminal carbon. In particular, alkyl is, for example, methyl, ethyl, n-propyl, isopropyl, n-butyl, isobutyl, or tert-butyl.

halo-C₁-C₇-alkyl may be linear or branched and in particular comprises 1 to 4 C atoms, for example 1 or 2 C atoms. Examples are fluoromethyl, difluoromethyl, trifluoromethyl, chloromethyl, dichloromethyl, trichloromethyl, 2-chloroethyl and 2,2,2-trifluoroethyl.

Halo or halogen is preferably fluoro, chloro, bromo or iodo, most preferably fluoro, chloro or bromo.

Alkenyl, being a radical or part of a radical, is a straight or branched (one or, if desired and possible, more times) carbon chain at least one double bond, its, for example, C₃-C₂₀-alkenyl (such as C₃-C₈alkenyl). In particular, alkenyl is, for example, allyl, which is unsubstituted or substituted as described herein.

Alkinyl, being a radical or part of a radical, is a straight or branched (one or, if desired and possible, more times) carbon chain containing at least one triple bond, and is, for example, C₃-C₂₀-alkinyl (such as C₃-C₇-alkinyl). In particular, alkinyl is, for example, propargyl, which is unsubstituted or substituted as described.

The term "C₃-₈cycloalkyl", as a radical or part of a radial, defines a cycloalkyl moiety with up to and including maximally 8, in particular up to and including maximally 6, carbon atoms. Said cycloalkyl moiety is for example mono- or bicyclic, in particular monocyclic, which may include one or more double and/or triple bonds. Embodiments include a cyclopropyl, cyclobutyl, cyclopentyl, cyclohexyl, or cyclooctyl

Heterocyclyl is a mono- or polycyclic, preferably a mono-, bi- or tricyclic-, most preferably mono-, unsaturated, partially saturated, saturated or aromatic ring system with preferably 3 to 20 (more preferably 5 to 10) ring atoms and with one or more, preferably one to four, heteroatoms independently selected from nitrogen, oxygen, sulfur, S(=O)₋ or S-(=O)₂₋ When the heterocyclyl is an aromatic ring it is also referred to as heteroaryl.

Alkoxy, being a radical or part of a radical, is, for example, C₁-C₂₀₋alkoxy (-O-C₁-C₂₀alkyl), preferably C₁-C₇-alkoxy (-O-C₁-C₇alkyl). In particular, alkoxy, is, for example, methoxy, ethoxy, n-propytoxy, isopropyloxy, n-butyloxy, isobutyloxy, see-butyioxy, tert-butyloxy, pentyloxy, hexyloxy and heptyloxy radicals.

Alkoxyalkyl may be linear or branched. The alkoxy group for example comprises 1 to 7 and in particular 1 or 4 C atoms, and the alkyl group for example comprises 1 tao 7 and in particular 1 or 4 C atoms. Examples are methoxymethyl, 2-methoxyethyl, 3-methoxypropyl, 4-methoxybutyl, 5-methoxypentyl, 6-methoxyhexyl, ethoxymethyl, 2-ethoxyethyl, 3-ethoxypropyl, 4-ethoxybutyl, 5-ethoxypentyl, 6-ethoxyhexyl, propyloxymethyl, butyloxymethyl, and 2-butyloxyethyl.

Alkanoyl, being a radical or part of a radical, is, for example, -C(=O)C₁-C₇alkyl. In particular, is, for example, acetyl [-C(=O)Me], propionyl, butyryl, isobutyryl or pivaloyl.

Aryl being a radical or part of a radical is, for example
C₆₋₁₀aryl, being a radical or part of a radical is preferably a mono- or polycyclic, especially monocyclic, bicyclic or tricyclic aryl moiety with 6 to 10 carbon atoms, preferably phenyl, indenyl, or naphthyl, most preferably phenyl.

The term arylalkyl refers to aryl-C₁-C₇-alkyl, wherein aryl is as defined herein and is for example benzyl, which is unsubstituted or substituted as described,

The term carboxyl refers to -CO₂H.

Aryloxy refers to a Aryl-O- wherein aryl is as defined above,

Preferred are selected from the group consisting of halo, nitro, G₁-C₇-alkyl, halo-C₁-C₇-alkyl, C₁-C₇-alkoxy, halo-C₁-C₇-alkoxy, C₁-C₇-alkoxy-C₁-C₇-alkyl, C₁-C₇-alkylC₁₋C₇-alkoxy and C₁-C₇-alkoxy-C₁-C₇-alkoxy.

The term "amine" in the expression "amine salt thereof" means tertiary amino of formula NR9RlGR11, secondary amine of formula NHR9RlOR or primary amino of formula NH₂R9, wherein R9, R10 and R11are, independently from one another, alkyl, aryl, cycloalkyl or heterocyclyl, as defined herein, preferably alkyl or cycloalkyl. The term "amine" is, for example, diphenylamine, diisopropylamine, dimethylamine, triethylamine, diisopropylethylamine, dicyclohexylamine, t-butylamine, n-butylamine or cyclohexylamine, in particular, t-butylamine, n-butylamine or cyclohexylamine.

Bonds with the asterisk (*) denote point of binding to the rest of the molecule.

In formulae above the term " 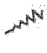 " represents a covalent bond, which comprises an (*E*) stereoisomer as well as a (*Z*) stereoisomer of the respective double bond.

The term "one-pot" "or "one-pot process" that in a series of reactions, each reaction product is provided for the next reaction without isolation and purification. The one-pot processes defined herein encompass not only a series of reactions conducted in a single reaction vessel, but also a series of reactions conducted in a plurality of reaction vessels (e.g., by transferring the reaction mixture from one vessel to other) without isolation and and purification. Preferably, the one-pot process is conducted in a single reaction vessel,

The term "step-wise process" means that in a series of reactions, each reaction product is provided for the next reaction with isolation and optionally purification.

The term "work-up" means the work of isolation and/or purification which is carried out once the reaction is finished.

As used herein, the term "room or temperature°' means a of from 15 to 30 °C, such as of from 20 to 30 °C, such as of from 20 to 25 ºC.

As used herein, the term "in-situ" refers to the capability of forming an azide of formula R7R8MN₃, wherein M is aluminium and R7 and R8 are as described herein, the addition of metal salt of hydrazoic acid to a reagent of as defined herein.

The term "organoaluminium azide" refers to a reagent is of formula R7R8MN₃, wherein M is aluminium and R7 and R8 are as defined herein.

The term "organoalumium halide" refers to a reagent is of formula R5AIX2 or as defined herein.

The compounds of the present invention can possess one or more asymmetric centers.

Salts are especially pharmaceutically acceptable salts or generally salts of any of the intermediates mentioned herein, where are not excluded for chemical the skilled person will readily understand. They be formed where salt forming groups, such as basic or acidic groups, are present that can exist in dissociated form at least partially, e.g. in a pH range from 4 to 10 in aqueous solutions, or can be isolated especially in solid, especially crystalline, form.

Such salts are formed, for example, as acid addition salts, preferably with organic or inorganic acids, from compounds or any of the intermediates mentioned herein with *a* basic nitrogen atom (e.g. imino or amino), the pharmaceutically acceptable salts. Suitable inorganic acids are, for example, halogen acids, as hydrochloric acid, sulfuric or phosphoric acid, Suitable organic are, for example, carboxylic, phosphonic, sulfonic or sulfamic acids, for example acetic acid, propionic acid, lactic acid, fumaric acid, succinic acid, citric acid, amino acids, such as glutamic acid or aspartic acid, maleic acid, hydroxymaleic acid, methylmaleic acid, benzoic acid, methane- or ethane-sulfonic acid, ethane-1 1,2-disuffonic acid, benzenesulfonic acid, 2-naphthalenesulfonic acid, 1,5-naphthalene-disulfonic acid, N-cyclohexylsuffamic N-methyl-, N-ethyl- or N-propyl-sulfamic acid, or other organic protonic acids, such as ascorbic acid,

In the presence of negatively charged radicals, such as or sulfo, salts may also be formed with bases, e.g. metal or ammonium salts, such as alkali metal or alkaline earth metal salts, for example sodium, potassium, magnesium or calcium salts, or ammonium salts with ammonia or suitable organic amines for example triethylamine or tri(2-hydroxyethyl)amine, N-ethyl-piperidine, N,N'-dimethylpiperazine, n-butylamine, phenylethylamine, dicyclohexylamine or cyclohexylamine.

When a basic group and an acid group are present in the same molecule, any of the intermediates mentioned herein may also form internal salts.

For isolation or purification purposes of any of the intermediates mentioned herein it us also possible to use pharmaceutically unacceptable salts, for example our perchlorates.

In view of the close relationship between the compounds and intermediates in free form and in the form of their salts, including those salts that can be used as intermediates, for example in the purification or identification of the compounds or salts thereof, any reference to "compounds", "starting materials" and "intermediates" hereinbefore and hereinafter is to be understood as referring also to one or more salts thereof or a mixture of a corresponding free compound, intermediate or starting material and one or more salts thereof, of which is intended to include also any solvate or salt of any one or more of these, as appropriate and expedient and if not explicitly mentioned otherwise, Different crystal forms may be and then are also included.

Where the plural form is used for compounds, starting materials, intermediates, salts, pharmaceutical preparations, diseases, disorders the like, this is intended to mean one (preferred) or more single compound(s), salt(s), pharmaceutical preparation(s), disease(s), disorder(s) or the like, where the singular or the indefinite article ("a", "an") is used, this is not intended to exclude the plural, but only preferably means "one".

The term if not defined specifically, is to be understood both as the free acid and as a salt thereof, especially a pharmaceutically acceptable salt thereof. Valsartan , or a pharmaceutically acceptable salt thereof, can, e.g., be prepared in a manner known per se, for example as described in WO2004/026847, in WO2005/014602 and in US5,399,578.

Preferred salts forms include acid addition salts. The compounds having at least one acid group (e.g., COOH or 5-tetrazolyl) can also form salts with bases, Suitable salts with bases are, e.g., metal salts, such as alkali metal or alkaline earth metal salts, e.g., sodium, potassium, calcium or magnesium salts, or salts with ammonia or an organic amine, such as morpholine, thiomorpholine, piperidine, pyrrolidine, a mono-, di- or tri-lower alkylamine, e.g., ethyl-, tert-butyl-, diethyl-, diisopropyt-, triethyl-, tributyl- or dimethylpropylamine, or a mono-, di- or trihydroxy lower alkylamine, e.g., mono-, di- or tri-ethanolamine. Corresponding internal salts may furthermore be formed. Salts which are for pharmaceutical uses but which can be employed, e.g., for the isolation or purification of free compounds l or their pharmaceutically acceptable are also included, Even more preferred salts are, e.g., selected from the mono-sodium salt in amorphous form; di-sodium salt of Valsartan in amorphous or crystalline form, especially in hydrate form, thereof. Mono-potassium salt of Valsartan in amorphous form; dipotassium salt of Valsartan in amorphous or crystalline form, especially in hydrate form, thereof.

Calcium salt of Valsartan in crystalline form, especially in hydrate form, primarily the tetrahydrate thereof; magnesium of Valsartan in crystalline form, especially in hydrate form, primarily the hexahydrate thereof; calcium/magnesium mixed salt of in crystalline form , especially in hydrate form; satt of in crystalline form, especially in hydrate form; *bis*-dipropyl ammonium salt of tun crystalline form, especially in hydrate form; *bis*-dibutylammonium salt of Valsartan in crystalline form, especially in hydrate form, the hemihydrate thereof; mono-L-arginine salt of in amorphous form; sa!t of in in amorphous form; mono-L-lysine salt of Valsartan in amorphous form; *bis*-L. lysine salt of Valsartan in amorphous form, Most preferably, Valsartan is used as the free acid.

### Abbreviations:

- AB: AB system
- AS-gem: AB geminal system
- ar.: aromatic
- arom.: aromatic
- arom-H: aromatic-H
- ar-H: aromatic-H
- ar-CH: aromatic-CH
- al-CH: aliphatic-CH
- all.: aliphatic
- ali.-CH: liphatio-CH
- δ: chemical shift
- Bn: benzyl
- Boc: tert-butoxycarbonyl
- BOC₂O: di-tert-butyl carbonate
- brm: broad multiplet
- br. sign.: broad signal
- or. s.: broad signal
- br. rune: broad multiplet
- br. mult.: broad multiplet
- cat,: catalytic amount
- compel, m: complex multiplet
- compl. mull. ; copl. m.: complex multiplet
- cpl. m.: complex multiplet
- Cbz: benzyl carbamate
- Cbz-Cl: enzyl chloroformate
- d: doublet
- dd: of doublet
- DCM: dichloromethane
- de: diastereomeric excess
- DMF: N,N-dimethylformamide
- DMSO: dimethylsulfoxide
- Et: ethyl
- EtOAc: ethyl acetate
- ETOH: ethanol
- FeCI₃: iron (lll) chloride
- FTIR: fourier transform infrared spectroscopy
- h: hour(s)
- HCl: hydrochloric acid
- ¹HNMR: proton nuclear magnetic resonance
- HPLC: high performance liquid chromatography
- i-Pr: isopropyl
- lR: infrared
- L: litre
- LCMS: liquid chromatography-mass spectrometry
- LRMS: low resolution mass spectroscopy
- M: molarity
- m/e: mass-to-charge ratio
- Me: methyl
- mg: milligram
- MgSO₄: magnesium sulfate
- m: multiple
- min: minute(s)
- mL: millilitre
- mmol(s): millimole(s)
- mol(s): mole(s)
- monosub.: monosubstituted
- m.p.: melting point
- MS: spectrometry
- mult. d: multiplet of doublets
- NaOH: sodium hydroxide
- NaOCI: sodium hypochlorite
- nm: nanometre
- NMR: nuclear magnetic resonance
- oct.: octet
- Pd/C: palladium on carbon
- Ph: phenyl
- ppm: parts per million
- psi: pounds per square inch
- RT: room temperature
- RuCl₃: ruthenium (III) chloride
- s: singlet
- SiO₂: silica gel
- sev. brm: several broad multiplets
- sev. dd: several doublets of doublets
- t: triplet
- temp.: temperature
- TBME: tertbutylmethylether
- TEMPO: 2,2,6,6-tetramethyf-l-piperidinyloxy
- TFA: trifluoroacetic acid
- THF: tetrahydrofuran
- TLC: thin layer chromatography
- Tos-: tosyl
- t_{R}: retention time
- vol.: volume
- wt.: weight

### Experimental:

The following terms are used herein below.
"Methylester-nitrile" = (*S*)-2-[(2'-cyano-biphenyl-4-ylmethyl)-pentanoy!-amino]-3-methyl-butyric acid methyl ester
"Benzyiester-nitrile" = (S)-2-[(2'-Cyano-biphenyl-4-ylmethyl).pentanoyl-amino]-3-methyl-butyric acid benzyl ester (described in *Organic Process Research & Development* **2007**, 11, 892)
°'Acid-nitrile" = (*S*)-2-[(2'-cyano-biphenyl-4-yfmethyl)-pentanoyl-amino]-3-methyl-butyric acid (described in Chinese of Pharmaceuticals 2001, 32(9), 385 and in EP1533305A1)
Valsartan = (*S*)-3-Methyl-2-{pentanoyl-[2'-(1H-tetrazol-5-yl)-biphenyl-4-ylmethyl]-amino}-butyric acid.

### Reference Example I: Preparation of "acid-nitrile" via hydrogenation of "benzylester-nitrile" with Pd/C

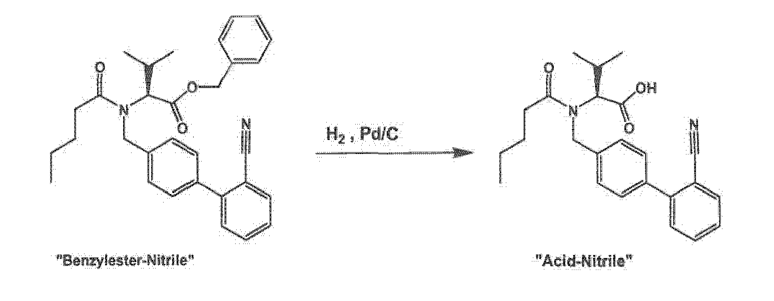

40 g (84 mmol) of "enzylester-Nitrile" are dissolved in 400 ml of dry ethanol are hydrogenated by addition of Pd/C 10 wt. % (Engelhard 4505) at 0.1 bar and 20-25 ° C in a shaking apparatus. After 1 hour the theoretical hydrogen up take is complete. For work up the catalyst is filtered and washed with additional 100 ml ethanol. The filtrate is evaporated in vacuum and dried in high vacuum to give a colourless oil.
¹H-NMR: (400 MHz, CDCI₃); δ_{H} (ppm):
67-0.70 (6H, 2dd, 2x -CH₃)_{1,} 0.78 (3H, d, -CH₃), 1.06-1.18 (2H, m, -CH₂-), 1.40-1.48 (2H, m, -CH₂-), 2.18-2.33 (2H, m, -CH₂-C=O), 2.50-2.58 (1H, m, -CH-), 3.37-3.50 (1H, d, -N-CH-COOH), 4.21 (1H, d, N-CH₂-Ph-), 4.63 (1H, d, N-H₂-Ph), 7.09 (2H, d, ar.-H), 7.24 (1H, t, ar.-H), 7.28 (1H, d, ar.-H), 7.34 (2H, d, ar.-H), 7.42 (1H,t, ar.-H).7.53 (1H, d,ar.-H)
MS: [M+H]⁺ = 393
IR: FTIR microscope in transmission [cm⁻¹]
broad -COOH, 3600-2400, 3064, 3030 (CH, ar-H), 2962,2932,2873 (ali.-CH), 2224(CN), 1735 (C=O), 1650 (C=O amide), 1650,1613 (ar.), 1478,1469,1446(ar), 1410,1390, 1373, 1354, 1271, 1203, 1169, 1131 , 1106, 1047, 1026, 1005, 967, 941, 850, 823 (CH-para), 765 (CH-ortho), 692

### Example II: Preparation of "acid-nitrile" via cleavage of the benzylester of "benzyl-nitrile" with diethylaluminium chloride

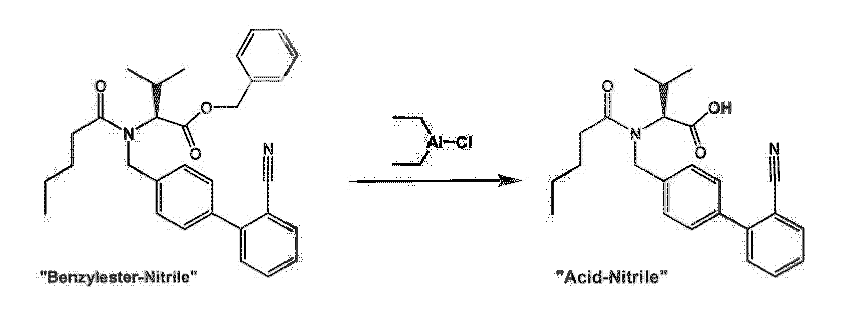

To solution of 2.41 g (5 mmol) of "Benzylester-Nitrile" in 10 ml of dry toluene is added via a syringe a 1.8 molar solution of diethylaluminium chloride (12.5 ml, 22.5 mmol) in toluene at room temperature under nitrogen and stirring. The addition is exotherm. After complete addition of the Et₂AICI solution the reaction mixture is warmed up to 50 °C. After 2 hours, the reaction mixture is cooled to 0 °C and is then quenched by slow addition of 20 ml of 2 molar hydrochloric acid. The quench reaction is quite exotherm and gas evolution is observed. The phases are separated and the organic phase is washed with 3 x 20 ml of water. The organic phase is evaporated in vacuum to give an oil. Spectroscopic data are the same as in Example 1,

### Reference Example III: Preparation of valsartan via a one-pot process with Diethylaluminium Azide

### Preparation of the Diethylaluminium azide reagent:

A dry 250ml flask under Argon is charged with 6.82 g (105 mmol) dry sodium azide. To the solid is added via a syringe under stirring a 2.7 molar solution (38.9 ml), 105 mmol of diethyl aluminium azide in xylene (isomeric mixture) during 1 hour. The white suspension is stirred at room temperature overnight. After this time, the suspension, containing solid NaC1 and diethylaluminium azide, is then ready for use.

### Cleavage of benzylester-nitrile to "Acid-Nitrile" and cycloaddition with Diethylaluminiumazide

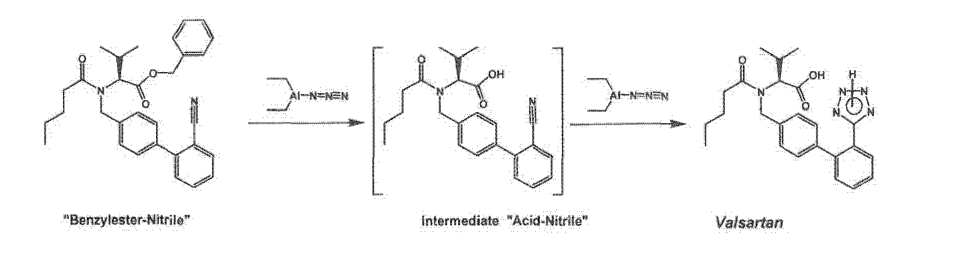

The above-prepared diethylaluminium azide suspension is warmed up in the same flask to 80 °C under stirring and under nitrogen. At this temperature, it is added slowly (45 min) via a dropping funnel a solution of 14.48 g (30 mmol) of "benzylester-nitrile" in 50 ml of xylene (isomeric mixture). After complete addition, HPLC analysis shows full conversion of the starting material to the "acid-nitrile". The internal temperature is then increased to 100°C. After stirring for 3.5 hours at this temperature, the internal temperature is further increased to 110°C. The reaction mixture is stirred for additional 4 hours at at 110°C and then heating is stopped and stirring is continued overnight at room temperature. Next, the suspension is cooled to 0 °C. To the suspension is then slowly added (1 hour) a mixture of an aqueous solution of sodium hydroxide (9 g, 225 mmol) in 50 ml of water and 15.52 g (225 mmol) of sodium nitrite. During the quenching the internal temperature is kept at 0ºC. Additional 2 molar aqueous sodium hydroxide solution (40 ml) and isopropyl acetate (50 ml) is added, to give a 3-phasic clear solution. The phases are separated, and the organic phase is extracted with water. The combined aqueous phases are neutralized with concentrated HCI and finally the pH is adjusted to 1.8. The product is extracted with ethyl acetate. The combined organic phases of the extractions are combined and evaporated in vacuum to give a slightly yellowish oil. The oily residue is crystallized from cyclohexane to give after filtration, and drying in a vacuum oven, valsartan, Spectroscopic data are the same as in Example IV,

**Example IV:** Preparation of Valsartan: via "one-pot process" with Diethylaluminium Chloride (for ester cleavage) and Diethylaluminium Azide (for the cycloaddition reaction) which is generated in-situ by reacting with Diethylaluminium Chloride and Sodium azide.

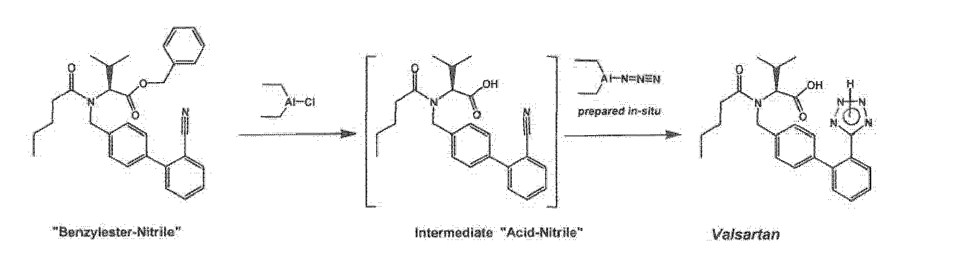

60 g of a solution of (S)-2-[(2'-Cyano-biphenyl-4-ylmethyl)-pentanoyl-amino]-3-methyl-butyric acid benzyl ester ("Benzylester-Nitrile") in xylenes (67.1 mmol) is mixed at 0°C with 33 g diethylaluminiumchloride (265.5 mmol) and stirred for 1 h, allowing the mixture to warm up to max. 50°C by intermittent cooling, 8.82 g of sodium azide (134.3 mmol) are added and the mixture is heated to 110°C. After 4 h, the reaction mixture is carefully transferred to a flask containing 201.6 g of 12 (w/w) % aqueous NaOH, while the temperature of the mixture is allowed to rise to 50°C. The upper organic phase is discarded, and the aqueous phase is washed with 60 g of xylenes.

An oily phase containing the product is separated and used for further work-up. It is dissolved in 120 g of water and 0.94 g of sodium nitrite (13.6 mmol) are added. Then, 41.5 g of 32% hydrochloric acid are added under cooling to 5°C. The resulting suspension is filtered on a Büchner funnel and washed with 100 ml of a 0.5% hydrochloric acid. The filter cake is dried in vacuum at 40-50°C to provide (S)-3-Methyl-2-{pentanoyl-[2'-(1H-tetrazol-5-yl)-biphenyl-4-ylmethyl]-amino}-butyric acid.
¹H-NMR (CDC1₃. 400 MHz, ppm): mixture of rotamers δ = 0.90-1.01 (9 H, m), 1.40 (2 H, m), 1.67 (2 H, m), 2.52 (2 H, m), 2.61 (1 H, m), 3.65 (1 H, d_{broad}), 4.05 and 4.29 (1 H, 2 d, ³J = 15.6 Hz), 4.89 and 5.16 (1 H, 2 d, ³J = 15.5 Hz), 7.12-7.14 (4 H, m), 7.41-7.60 (3 H, m), 7.87-7.94 (1H, m) ppm.

### Example V: Esterification of intermediate "acid-nitrile" with 3-methyl-1-(p-tolyl)-triazene

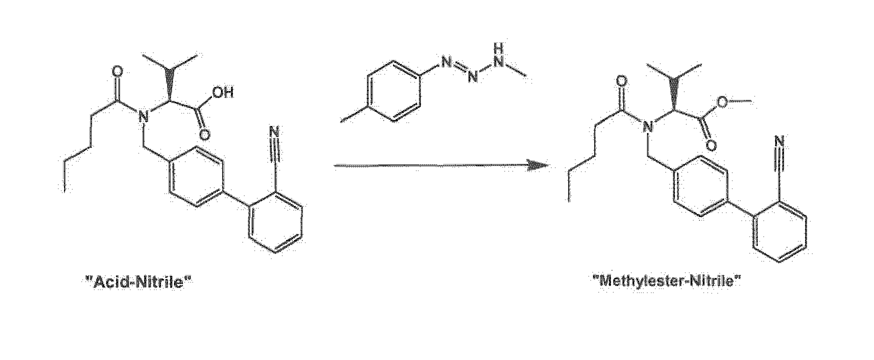

9.8 g (65.56 mmol) of 3-methyl.1-(p.tolyl)-triazene are dissolved in 100 ml of dichloromethane. To this solution is added via a dropping funnel a solution of 24.4g (59.6 mmol) of "acid-nitrile" dissolved in 200 ml of dichloromethane at room temperature during 45 minutes under stirring. After 2 hours, the reaction mixture is treated with 100 ml of 1 molar hydrochloric acid. The organic phase is washed with water, dried over sodium sulfate and evaporated in vacuum to give a slightly yellow oil, which is pure according to HPLC and H-NMR analysis.
¹H-NMR: (400 MHz, CDC1₃); δ_{H} (ppm), rotamer mixture (6: 4) at room temperature. 0.82-0.95 (5H, m, -CH3, 60 %), 0.95-1.03 (4H, m, -CH₃, 40%), 1.25-1.37 (1H, m,-CH-, 40%), 1.40-1.49 (1H, m, -CH-,60%), 1.60-1.70 (1H, m, -CH-, 60%), 1.70-1.82 (1H,m,-CH-, 40%), 2.22-2.65 (3H, br, copl. m, -CH- and -CH₂-), 3.37 (s, -OCH₃, 40%), 3.45 (s, - OCH₃, 60%), 4.07 (0.5 H,40%, d, N-CH-CO₂Me), 4.27 (0.5 H, 40%, d, N-CH₂-Ph-Ph), 4.69 (2H, 60%, ab, dd, -N-CH₂-Ph-Ph), 4.98 (1H, 60%, d, N-CH-CO₂Me), 5.08 (0.5H, 40%, -N-CH₂-Ph-Ph), 7.25-7.34 (3H, m, ar.-H), 7.40-7.58 (4H, m, ar.-H), 7.60-7.69 (1H, m, ar.-H), 7.72-7.80 (1H, m, ar.-H).
MS: [M+H]⁺=407, [M+NH₄]⁺ = 424
IR: FTIR microscope in transmission [cm⁻¹]
3064, 3029 (CH, ar-H), 2980,2933,2873 (all.-CH), 2224(CN), 1739 (C=O), 1652 (C=O amide), 1597,1561, 519,1504,1478,1464,1435 (ar), 1408,1372,1265,1204 (C-O-ester) 1169,1133,1106,1007, 974, 946, 888, 824 (CH-para), 765 (CH-ortho).

**Example VI:** Preparation of Valsartan: via "one-pot process" with Diethylaluminium Chloride (for ester cleavage) and Diethylaluminium Azide (for the cycloaddition reaction) is generated in-situ by reacting the excess of Diethylaluminium Chloride with Sodium azide.

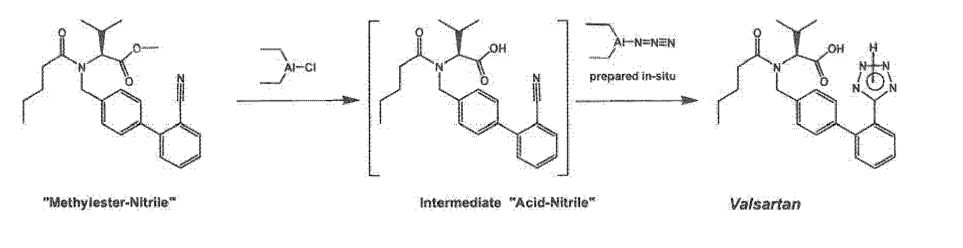

In an argon filled three necked flask, 2.4 g (3.00 mmol) of a solution of (S)-2₋[(2'-cyanobiphenyl-4-ylmethyl)-pentanoyl-amino]-3-methyl-butyric acid methyl ester in xylenes (3.00 mmol) is diluted with 5 ml of dry xylene (40 mmol). The mixture is heated to 110°C and 1.94 ml of diethylaluminium chloride (15 mmol) are added. The mixture is stirred at 110°C. After 60 minutes, 0.59 g of sodium azide are added as a solid in one portion. The mixture is stirred at 110°C. After 44h, the reaction mixture is allowed to cool to room temperature and transferred into a three necked flask containing 20 eq of 10% (w/w) aqueous sodium hydroxide (24.0 g, 60 mmol) while the temperature is maintained below 30°C. The resulting biphasic mixture is transferred into a separation funnel and the upper xylenic phase is discarded. The aqueous phase is washed with 6.8 ml of xylenes (55 mmol). Sodium nitrite (0.45 g, 6.6 mmol) is dissolved in the basic aqueous solution. The mixture is added carefully to an emulsion of 7.99 g of 37% aqueous hydrochloric acid (81 mmol) and 10 ml ethyl acetate (102 mmol) while the temperature is maintained below 5°C in an ice-bath. The phases are separated and the aqueous phase is extracted with 10 ml ethyl acetate (102 mmol). The combined organic phases are washed with 10 ml H₂O (555 mmol). The solvent is removed under vacuum and 20 ml of xylenes (160 mmol) are added and the mixture is again concentrated to dryness. The crude oily product is dissolved in 15 ml EtOAc (153 mmol), concentrated to approx. 5 ml and 2 ml cyclohexanes (21 mmol) are added dropwise until a white precipitate does just redissolve at 60 °C. The mixture is placed in a fridge at 8 °C. After 16 h, an off-white precipitate is collected by filtration. The product is washed with cold ethyl acetate /cyclohexane 1:1 and dried under vacuum to give (S)-3-Methyl-2-{pentanoyl-[2'-(1H-tetrazol-5-yl)-biphenyl-4-ylmethyl]-amino}-butyric acid (Valsartan). 1 H-NMR (CDC13, 400 MHz, ppm): main rotamer δ= (0.94-1.05 (9 H, m), 1.40-1048 (2 H, m), 1.72-1.79 (2 H, m), 2.61-2.65 (2 H, m), 2.69-2.78 (1 H, m), 3.36 (1 H, d, JH,H = 11.1 Hz), 4.21 (1 H, d, JH,H = 14.8 Hz), 5.00 (1 H, d, JH,H = 14.8 Hz), 7.19-7.25 (4 H, m), 7.47-7.50 (1 H, m), 7.52-7.56 (1 H, m), 7.59-7.63 (1 H, m), 8.07-8.09 (1 H, m).

### Reference Example V: Cleavage of Phthalic acid bisallylester with Me₂A1-C1

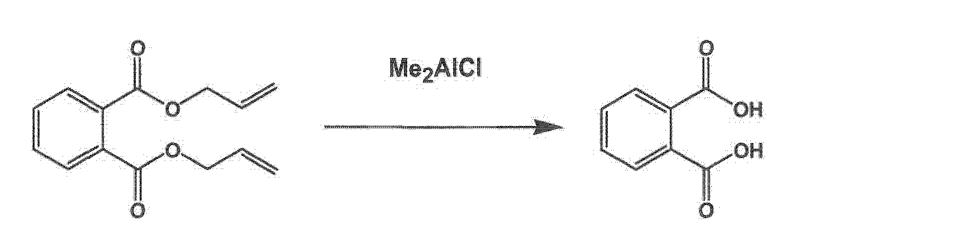

246 mg (1mmol) of diallyl phthalate are dissolved in 10 ml of dry toluene under nitrogen. The solution is heated to reflux (110 °C) and 2.1 ml of a 1 molar solution of dimethyl aluminium chloride (2.1 equiv.) are added via a syringe under nitrogen. The reaction mixture is stirred over night at 110 °C. The reaction mixture is cooled to room temperature, quenched with 15 ml of 2 molar HCI and extracted with 7 x 15 ml of ethyl acetate. The combined organic phases are extracted with 2 x 12 ml of saturated sodium bicarbonate solution. The combined basic, aqueous phase is adjusted to pH 1 with 10 ml of diluted sulfuric acid. The acidic aqueous is again extracted with 7 × 15 ml of ethyl acetate and the combined organic phase are washed with brine, dried over sodium sulfate, filtered and in vacuum to phthalic acid. ¹H-NMR and LC-MS confirm the structure of phthalic acid.

### Reference Example Vl: Cleavage of benzoic acid benzylester with MeAlCl₂

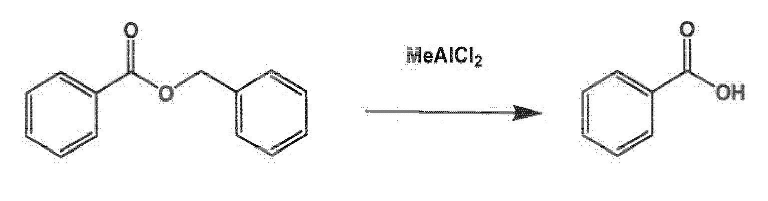

212 mg (1 mmol) of benzoic acid benzyl ester are dissolved in 10 ml of dry toluene under nitrogen atmosphere. The reaction mixture is heated up to reflux and 1.05 ml (1.05 equv.) of methyl aluminium dichloride in hexane solution (1 molar) is in 1 portion, Heating is continued over night. The reaction mixture is cooled to room temperature and quenched onto 15 ml of 2 M hydrochloric acid. The reaction mixture is extracted with 4 × 15 ml of dichloromethane. The combined organic phases are extracted 2 × 12 ml of saturated sodium bicarbonate solution. The combined aqueous basic phases are then adjusted to pH 1 with 2 ml of conc sulfuric acid. The acidic aqueous phase is extracted with 4 × 15 ml of dichloromethane the combined dichloromethane extracts are then washed with brine, dried over sodium sulfate, filtered and evaporated in vacuum to give benzoic acid. ¹H-NMR and LC-MS confirm the structure of benzoic acid (comparison with reference material).

**Example lX:** Preparation of Valsaran: via "one-pot process" with Diethyl-aluminium Chloride (for ester cleavage) and Diethylaluminium Azide (for the cycloaddition reaction)

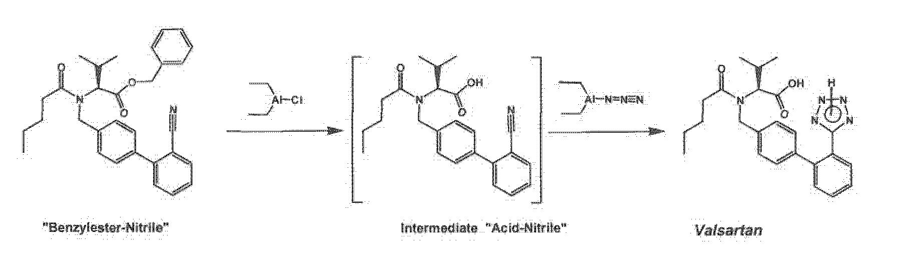

### Preparation of the diethyl-Al-azide reagent:

A 250 ml flask is charged under argon with 3.9 g (65 mmol) of NaN₃ and 10 ml of dry xylene is added. To this suspension is added, at room temperature under stirring, 33 ml of a 2 molar solution (66 mmol) of diethylaluminium chloride during 15 min. The is then stirred for further 5 hours at room temperature and it is then ready for use. The diethyl aluminium azide is in solution but the solid NaCl is in a suspension.

### Cleavage of benzylester-Nitrile to "Acid-Nitrile" and cycloaddition with Diethylaluminium azide

A separate 100 ml flask is charged with 9.64 g(20 mmol) of (benzylester-nitrile) which is then dissolved at room temperature under argon and stirring with 20 ml of dry xylene. To the solution is added at room temperature under stirring 33 ml of a 2 molar solution of diethylaluminium chloride. After stirring at room temperature over night additional 17 ml of 2 M diethylaluminium chloride is added. After stirring for additional 3 hours at room temperature, the solution is then added via a dropping to the 250 ml flask containing the above diethylaluminium solution at room temperature.

The reaction mixture is then heated to reflux (external temp. 150 °C). After 14, the reaction mixture is cooled to room temperature and worked up by quenching the reaction mixture to a solution of sodium nitrite (4.14 g) in 25 ml of water and 6.7 g of a sodium hydroxide solution (30 %, aqueous) under vigorous stirring under argon and external cooling. The reaction flask is rinsed with 10 ml of water and 20 ml of xylene. The resulting suspension is then adjusted to pH 2-3 by slow addition of 25 ml of a 5 M hydrochloric acid under stirring. By further addition of 60 ml of 5 M HCI, until pH 0 is reached, a slightly turbid yellowish solution is obtained. The phases are separated and the organic phase is washed with 4 × 25 ml of water. The organic phase is evaporated in vacuum to give a yellow foam. The yellow residue is dissolved in 50 ml of EtOAc. The product is extracted to the aqueous phase by 50 ml and 20 ml of sodium bicarbonate solution (8%, aqueous), The aqueous phase contains the desired product. The product containing basic aqueous phase is adjusted to pH 0-1 by addition of 25 ml of 5 molar hydrochloric acid and extracted to 50 ml of EtOAc. The organic phase is washed with water (2 × 25 ml), evaporated in vacuum to give a yellowish foam.The foam is dissolved for crystallization in 30 ml of ethyl acetate and treated with additional 5 ml of heptane. Crystallization in the fridge at 7 °C occurs overnight. The crystal suspension is further diluted at 0 °C by addition of 100 ml of heptane. Filtration, washing and drying at 40 °C in vacuum gives valsartan (as confirmed by ¹H-NMR analysis).

### Example x:Preparation of "Propargylester-Nitrile" from "Acid-Nitrite"

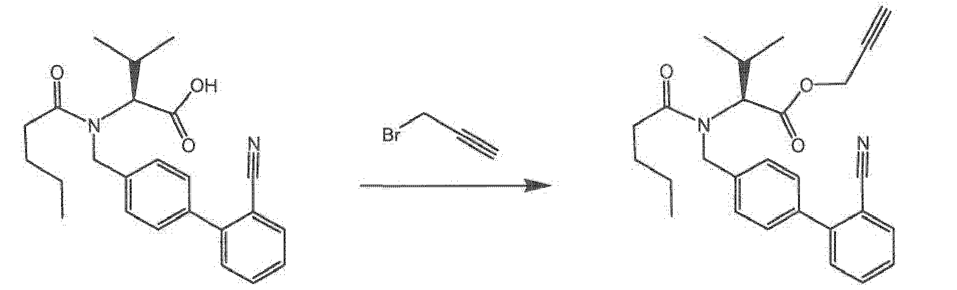

7.85 g (20 mmol) of "Acid-Nitrile" are dissolved in 100 ml of dry DMF. To this solution is added 3.36 (40 mmol) of solid sodium bicarbonate under stirring to give a slightly yellow suspension. To this suspension is added 8.9 ml (100 mmol) of propargyl bromide, dissolved in 100 ml of dry DMF. The reaction mixture is stirred at room temperature overnight. Additional 1ml (11.3 mmol) of propargyl bromide and 0.4 g (11.9 mmol) of sodium bicarbonate are added. After further stirring at room temperature for 4 hours, the reaction mixture is diluted with 200 ml of ethylacetate, followed by extraction of the organic phase with 3 × 250 ml of water and finally with 200 ml of brine. The organic phase is dried over MgSO4, filtered and evaporated and dried under high vacuum to give crude propargylic ester, which is pure enough for further reactions.
¹H-NMR: (400 MHz, d6-DMSO); δ_{H} (ppm), the product is a mixture of rotamers at room temperature:
0.75-0.87 (5H, br.m, -CH₃), 0.90-1.00 (4H, m, -CH₃), 1.15-1.27 (2H, br. m), 1.31-1.54 (2H, br.m), 2.15-2.25 (1H, br.m), 2.28-2,40 (3H, br.m), 2.52-2.65 (1H, br.m), 3.03 (1H, s, acetylen-H), 4.30-4.60 (4H, compl. m), 4.68-4.82 (2H, m), 7.24-7.98 (8H, sev. mpl., rotamer mix).
   MS: [M+H]⁺ = 431.2, (M+NH4)⁺ = 448.2
   lR- FTlR microscope in transmission [cm⁻¹]
3288, 3064, 3030 (CH, ar-H), 2962, 2933, 2873 (ali.-CH), 2224(CN), 2128 (CC-triple b.) 1745 (C=O, ester), 1652 (C=O, amide), 1597, 1478, 1469, 1445, 1408, 1389, 1374, 1354, 1267, 1234, 1193, 1167, 1129, 1024, 997, 973, 942, 824, 766.

### Example Xl: Preparation of "Cinnamylester-Nitrile" from "Acid-Nitrile"

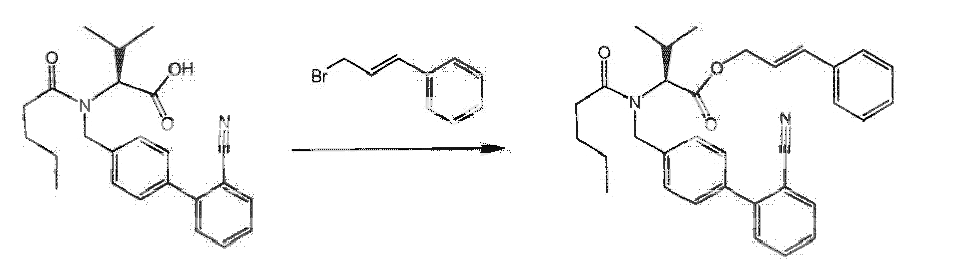

3.93 g (10 mmol) of "Acid-Nitrile" is dissolved in 50 ml of dry DMF. To this solution is added 1.68 (20 mmol) of solid sodium bicarbonate under stirring to give a slightly yellow suspension. To the suspension is added 5.85 g (50 mmol) of 3-bromo-1-phenyl-1 propene, dissolved in 50 ml of dry DMF. The reaction mixture is stirred at room temperature overnight. After 16 hours, the reaction mixture is diluted with 100 ml of ethylacetate, followed by extraction of the organic phase with 3 × 130 ml of water and finally with 100 ml of brine. The organic phase is dried over MgSO4, filtered, evaporated and dried under vacuum to give crude "cinnamylester-nitrile". The crude product was purified by column chromatography on silica gel (400 g) first with cyclohexane and then with cyclohexane/ethylacetate (8:1). The product containing fractions were again chromatographed by a second SiO₂ -column (360 g) with cylohexane/ethylacetate (5 : 1) to give the product as a colourless oil.
¹H-NMR: (400 MHz, CDCl₃); δ_{H}(ppm), the product is a ca. 2:1 -mixture of rotamers at room temperature:
0.84-1.02 (6H, compl. m., 2× -CH₃), 1.05 (3H, d, -CH₃), 1.40-1.50 (1Hm), 1.40-1.48 (2H, m, -CH₂-), 2.18-2.33 (2H, m, -CH₂-C=O), 2.50-2.58 (1H, m, -CH-), 3.37-3,50 (1H, d, -N-CH-COOH), 4.20 (1H, d, N-CH₂-Ph-), 4.63 (1H, d, N-H₂-Ph), 7,20-7.39 (5H, compl. m., ar.-H), 7.40-7.51 (4H, br.m., ar.-H), 7.60-7.67 (1H, m, ar.-H), 7.78 (1H. t, ar.-H)
MS: [M+H]⁺ = 509.3, (M+NH4)⁺ = 526.3, 527.3
lR: FTlR microscope in transmission [cm⁻¹
3061, 3028 (CH, ar-H), 2961, 2933, 2873 (ali.-CH), 2224(CN), 1737 (C=O, ester), 1653 (C=O, amide), 1495, 1478, 1466, 1447, 1408, 1388, 1356, 1299, 1264, 1195, 1168, 1130, 1109, 1027, 1004, 970, 944, 823, 765, 746, 693.

### Example Xll:Preparation of "Allylester-Nitrile"

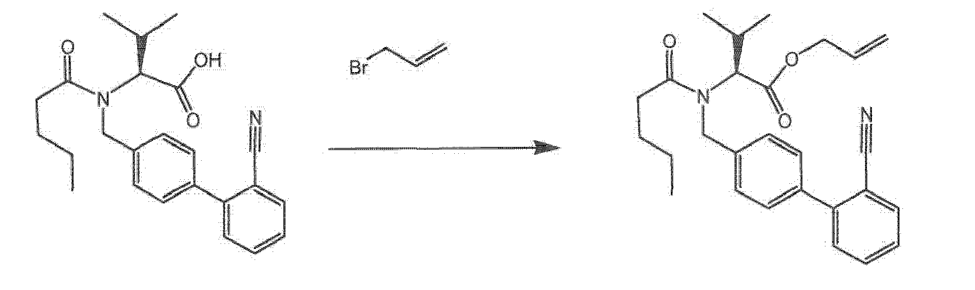

Compound "Allylester-Nitrite" is prepared according to analogous method to that described in the example above for the "Cinnamylester-Nitrile".

### Spectroscopic data of "Allylester-Nitrile":

¹H-NMR: (400 MHz, d6-DMSO)-, δ_{H} (ppm), the product is a ca. 2:1 -mixture of rotamers at room temperature:
0.75-0.85 (5H, br. m., -CH₃), 0.88-0.98 (4H, br. m., -CH₃), 1.1 5-1.25 (2H, br. m.,) 1.30-1.38 (1H, br. m.), 1.40-1.52 (2H, br. m), 1.53-1.66 (1H, br. m), 2.13-2.22 (1H, m), 2.28-2.40 (2H, m), 2.50-2.63 (1H, br. m), 4.16-4.40 (3H, br. m), 4.60 (1H. d), 4.68 (1H, d), 4.85 (1H, dd, AB)_{.} 5.20 (1H. q, olef.-H), 5.25 (1H, d, olef.-H), 5.68-5.83 (1H, br. m., olef.-H), 7.23 (rota. mix, d, ar.-H), 7.38 (rota.mix, d, ar.-H), 7.48 (rota.mix, d, ar.-H), 7.52-7.62 (3H, br. m, ar.-H), 7.78 (1H. q, ar.-H), 7.93(1H, t, ar.-H).
   MS: [M+H]⁺ =433.2.3, (M+NH4)⁺ = 450.3
   lR: FTlR microscope in transmission [cm⁻¹]
3065, 3029 (CH, ar-H), 2961, 2933, 2873 (ali.-CH), 2224 (CN), 1738 (C=O, ester), 1653 (C=O, amide), 1597, 1562, 1518, 1479, 1468, 1445, 1408, 1388, 1372, 1267, 1197, 1170, 1131, 1107, 1005, 991, 937, 822, 765.

### Example Xlll:Preparation of "Prenylester-Nitrile"

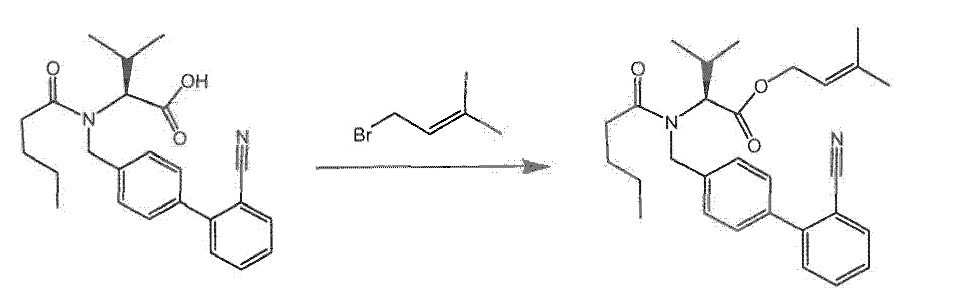

Compound "Allylester-Nitrile" is prepared according to analogous method to that described in the example above for the "Cinnamytester-Nitrile".

### Spectroscopic data of "Prenylester-Nitrile":

¹H-NMR: (400 MHz, CDCl₃); δ_{H} (ppm), the product is a mixture of rotamers at 27° C.
0.85-1.05 (9H, compl. m, 3x -CH₃), 1.25-1.30 (2H, m, -CH-), 1.55-1 1.70 (2H, m, and 3H, s, -CH₃),1.70-1,83 (1H, m, & 3H, s,-CH3), 2.20-2.68 (3H, br.m), 4.05-4.22 (1H, compl. m), 4.30-4.55 (3H, compl. m), 4.68 (1H_{,} d, AB), 4.8 (1H, d. AB), 4.88-4.98 (2H, m), 5.20-5.32 (2H, m), 7.32-7.38 (2H, m, ar.-H), 7.40-7.58 (5H, br.m., ar.-H), 7,62-7.71 (1H, br.m., ar.-H), 7.74-7.82 (1H, br.m., ar.-H).
   MS: [M+H]⁺ =461, (M+NH4)⁺ = 478
   IR: FTIR microscope in transmission [cm⁻¹]
3064, 3029 (CH, ar-H), 2962, 2933, 2873 (ali.-CH), 2224(CN), 1734 (C=O, ester), 1654 (C=O, amide), 1597, 1561, 1518, 1478 ,1445 ,1408 ,1384 ,1356 ,1267 ,1196 ,1168, 1130, 1108, 1047, 975, 942, 823, 765.

### Example XIV: Cleavage of "Prenylester-Nitrile" with methylaluminium dichloride

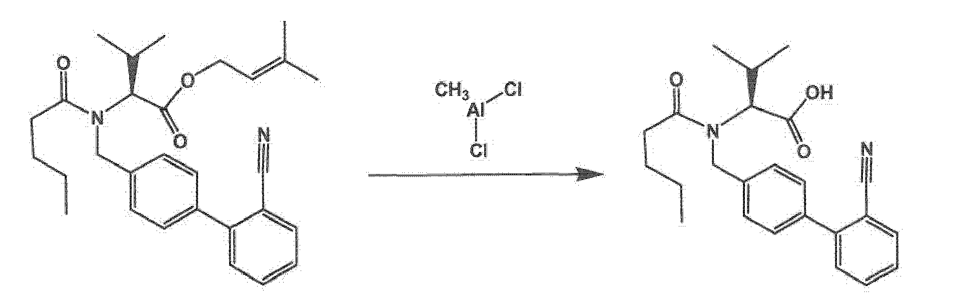

A 25 ml flask, dried with a heating gun under a flow of nitrogen, is charged with 460 mg (1 mmol) of "prenylester-nitrile" and 8 ml of dry toluene to get a yellow solution. To this solution is added dropwise a 1 molar n-hexane solution (3.5 ml , 3.5 mmol) of methylaluminium dichloride at room temperature to get a red suspension. The reaction mixture is heated to reflux over night (16 hours). The reaction mixture is cooled to room temperature and then quenched on 20 ml of 1 molar HCI, To the biphasic reaction mixture is added 20 ml of ethylacetate. The organic phase is separated and washed 3-times with 20 ml of water and with 20 ml of brine. The organic phase is dried with sodium sulfate, filtered and evaporated under vacuum to give the crude "acid-nitrile" as a brown oil. The crude is purified by column chromatograhy over 20 g of Si02 with a solvent mix of cyclohexane : ethylacetate : acetic acid (60 : 40 : 2). The product containing fractions are combined to give the "acid-nitrile" as a brown oil. The spectroscopic data of this material are identical with the data of "acid-nitrile" obtained in example above.

Propargylester-Nitrile, Cinnamylester-nitrile and Allylester-nitrile are by this method using different alkylaluminium halides, as defined above, to give the desired product "add-nitrile" in different yields,

## Claims

1. A process for the manufacture of a compound of formula (IV) or salt thereof, such as an amine salt thereof, 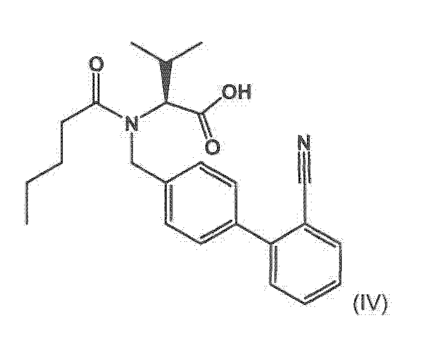 comprising
reacting a compound of formula (III), or salt thereof, 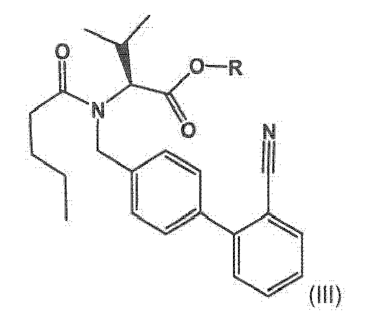 R is C₁₋₇alkyl, 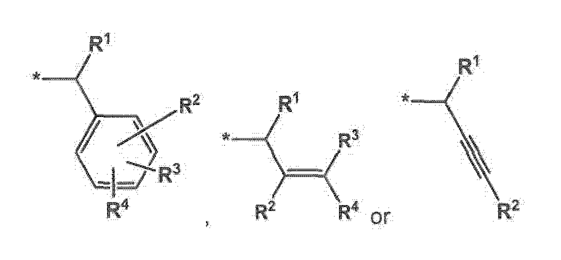 wherein R1 is hydrogen, C₁₋₇alkyl, C₁₋₇alkoxy, C₃₋₈cycloalkyl or C₆₋₁₀aryl, each unsubstituted or substituted by one or more, e.g. up to three, substituents selected from halo, nitro, C₁₋₇alkyl, halo- C₁₋₇alkyl, C₁₋₇alkoxy- C₁₋₇alkoxy, halo-C₁₋₇alkoxy,C₁₋₇alkoxy-C₁₋₇alkoxy, halo-C₁₋₇alkoxy-C₁₋₇alkoxy, C₁₋₇alkyl-C₁₋₇alkoxy, halo-C₁₋₇alkyl-C₁₋₇alkoxy; and
R2, R3 and R4 are the same or different form each other and are hydrogen, halo, nitro, C₁₋₇alkyl, C₁₋₇alkoxy, C₃₋₈cycloalkyl or C₆₋₁₀aryl, each unsubstituted or substituted by one or more, e.g. up to three, substituents selected from , halo, nitro, C₁₋₇alkyl, halo- C₁. ₇alkyl, C₁₋₇alkoxy-C₁₋₇alkyl, C₁₋₇alkoxy, halo-C₁-₇alkoxy, C₁-₇alkoxy-C₁-₇alkoxy, halo-C₁. ₇alkoxy-C₁₋₇alkoxy, C₁₋₇alkyl-C₁₋₇alkoxy, halo-C₁-₇alkyl-C₁₋₇alkoxy;
with an organoaluminiun halide of formula R5R6AIX or R5AIX₂
wherein R5 and R6 are, independently from one another, C₁-₇alkyl, C₃-C₈alkenyl ₈cycloalkyl or C₆₋₁₀aryl, preferably C₁₋₇alkyl, and X is halogen,
to obtain the compound of formula (lV).

2. A process for the manufacture of a compound of formula (I), or salt thereof, 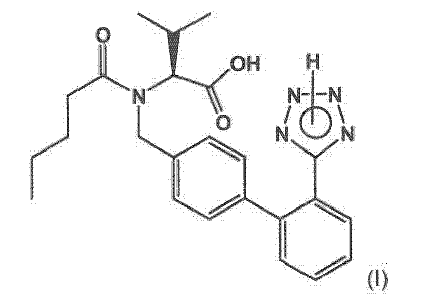 comprising
reaching a compound of formula (V), or salt thereof, 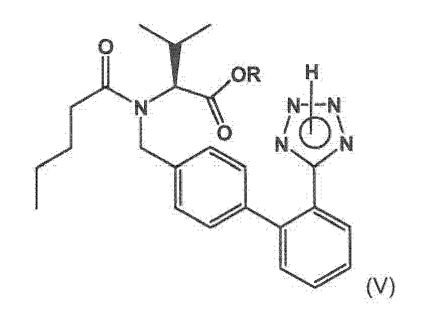 wherein R is C₁₋₇alkyl, 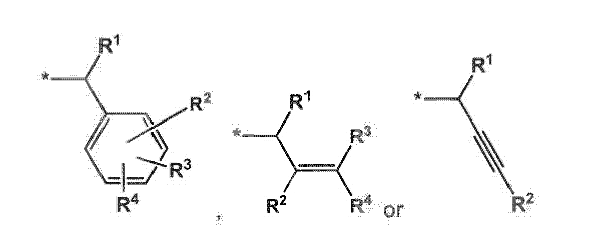 wherein R1 is hydrogen, C₁₋₇alkyl, C₁₋₇alkoxy, C₃₋₈cycloalkyl or C₆₋₁₀aryl, each unsubstituted or substituted by one or more, e.g. up to three, substituents selected from , halo, nitro, C₁₋₇alkyl, C₁₋₇alkyl, C₁₋₇alkoxy-C₁₋₇alkyl, C₁₋₇alkoxy, halo-C₁₋₇alkoxy, C₁₋₇alkoxy-C₁₋₇alkoxy, halo-C₁₋₇alkoxy-C₁₋₇alkoxy, C₁₋₇alkyl-C₁₋₇alkoxy, halo-C₁₋₇alkyl-C₁₋₇alkoxy; and
R2, R3 and 4 are the same or different form each other and are hydrogen, halo, nitro, C₁₋₇alkyl, C₁₋₇alkoxy, C₃₋₈cycloalkyl or C₆₋₁₀aryl, each unsubstituted or substituted by one or more, e.g. up to three, substituents selected from , halo, nitro, C₁₋₇a)ky), halo- C₁₋₇alkyl, C₁₋₇alkoxy-C₁₋₇alkyl, C₁₋₇alkoxy, halo-C₁₋₇alkoxy, C₁₋₇alkoxy-C₁₋₇alkoxy, halo-C₁₋₇alkoxy-C₁₋₇alkoxy, C₁₋₇alkyl-C₁₋₇alkoxy, halo-C₁₋₇alkyl-C₁₋₇alkoxy;
with an organoaluminiun halide of formula R5R6AIX or R5AIX₂
wherein R5 and R6 are, independently from one another, C₁₋₇alkyl, C₃-C₈alkenyl, C₃₋₈cycloalkyl or C₆₋₁₀aryl, preferably C₁₋₇alkyl, and X is halogen,
to obtain the compound of formula (|).

3. A step-wise process for the manufacture of a compound of formula (I), or salt thereof, 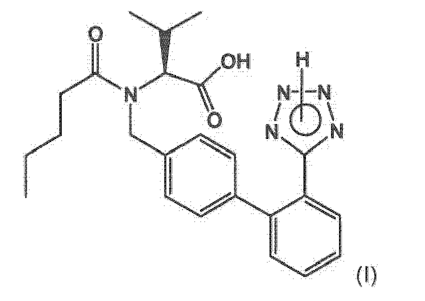 comprising the steps of
i) reacting a compound of formula (III), or salt thereof, 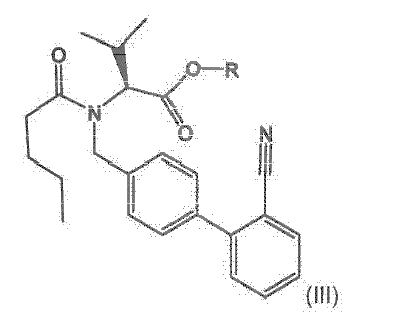 R is C₁₋₇alkyl, 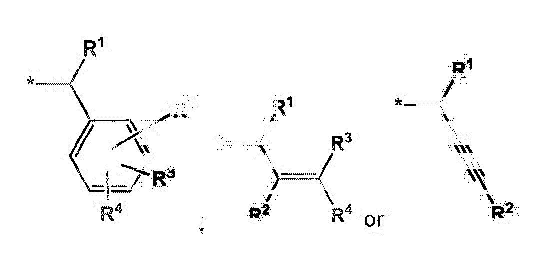 wherein R1 is hydrogen, G₁₋₇alkyl, C₁₋₇alkoxy, C₃₋₈cycloalkyl or C₆₋₁₀aryl, each unsubstituted or substituted by one or more, e.g. up to three, substituents selected from , halo, nitro, C₁₋₇alkyl, halo- C₁₋₇alkyl, C₁₋₇alkoxy-C₁₋₇alkyl, C₁₋₇alkoxy, halo-C₁₋₇alkoxy, C_{1- 7}alkoxy-CI-7alkoxy, halo-C₁₋₇alkoxy-C₁₋₇alkoxy, C₁₋₇alkyl-C₁₋₇alkoxy, halo-C₁₋₇alkyl-C₁₋₇alkoxy; and
R2, R3 and R4 are the same or different form each other and are hydrogen, halo, nitro, Cₗ-₇alkyl, C₁₋₇alkoxy, C₃₋₈cycloalkyl or C₆₋₁₀aryl, each unsubstituted or substituted by one or more, e.g. up to three, substituents selected from , halo, nitro, C₁₋₇alkyl, halo- C₁₋₇alkyl, C₁₋₇alkoxy-C₁₋₇alkyl, C₁₋₇alkoxy, halo-C₁₋₇alkoxy, C₁₋₇alkoxy-C₁₋₇alkoxy, halo-C₁₋₇alkoxy-C₁₋₇alkoxy, C₁₋₇alkyl-C₁₋₇alkoxy, halo-C₁₋₇alkyl-C₁₋₇a[koxy;
with an organoaluminiun halide of formula R5R6AIX or R5AIX₂
wherein R5 and R6 are, independently from one another, C₁₋₇alkyl, C₃-C₈alkenyl, C₃₋₈cydoalkyl or C₆₋₁₀aryl, preferably C₁₋₇alkyl, and X is halogen,
to obtain the compound of formula (IV), or salt thereof,
such as an amine salt thereof, 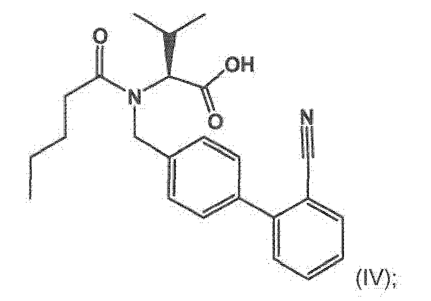
ii) treating the compound of formula (IV), or salt thereof,
such as an amine salt thereof,
with an azide reagent to provide compound of formula (I).

4. A one-pot process for the manufacture of a compound of formula (I), or salt thereof, 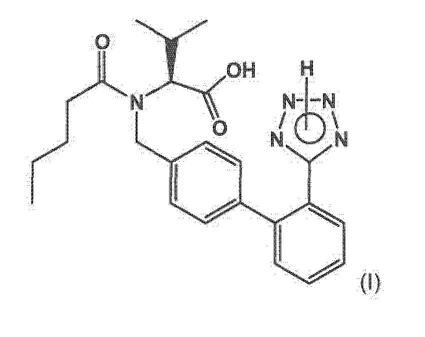 comprising
i) a compound of formula (III), or salt thereof, 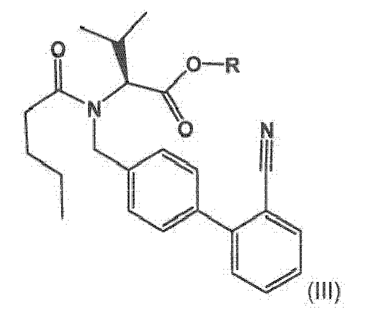 wherein R is C₁₋₇alkyl, 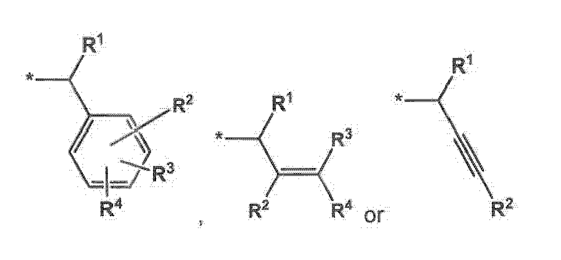 wherein R1 is hydrogen, C₁₋₇alkyl, C₁₋₇alkoxy, C₃₋₈cycloalkyl or C₆₋₁₀aryl, each unsubstituted or substituted by one or more, e.g. up to three, substituents selected from , halo, nitro, C₁₋₇alkyl, halo- C₁₋₇alkyl, C₁₋₇alkoxy-C₁₋₇alkyl, C₁₋₇alkoxy, halo-C₁₋₇alkoxy, C₁**.** ₇alkoxy-C₁₋₇alkoxy, halo-C₁₋₇alkoxy-C₁₋₇alkoxy, C₁₋₇alkyl-C₁₋₇alkoxy, halo-C₁₋₇alkyl-C₁. ₇alkoxy, and
R2, R3 and R4 are the same or different form each other and are hydrogen, halo, nitro, C₁₋₇alkyl, C₁₋₇alkoxy, C₃₋₈cycloalkyl or C₆₋₁₀aryl, each unsubstituted or by one or more, e.g. up to three, substituents selected from , halo, nitro, C₁-₇alkyl, halo- C₁₋₇alkyl, C₁₋₇alkoxy-C₁₋₇alkyl, C₁₋₇alkoxy, halo-C₁₋₇alkoxy, C₁₋₇alkoxy-C₁₋₇alkoxy, halo-C₁₋₇alkoxy-C₁-₇alkoxy, C₁₋₇alkyl-C₁₋₇alkoxy, halo-C₁₋₇alkyl-C₁₋₇alkoxy;
with an organoaluminiun halide of formula or R5AIX₂
wherein R5 and R6 are, independently from one another, C₁₋₇alkyl, C₃-C₈alkenyl, C₃₋₈cycloalkyl or C₆₋₁₀aryl, preferably C₁-₇alkyl, and X is halogen,
to obtain the compound of formula (IV), or salt thereof, such as an amine salt thereof, 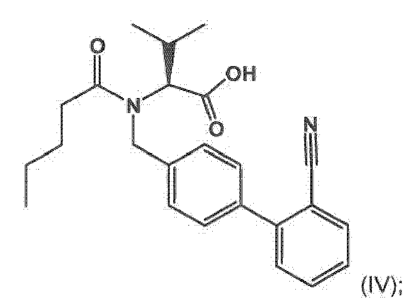
ii) adding to the resulting reaction mixture of step i) an azide reagent to obtain the compound of formula (I).

5. The process according to any one of the preceding claims, wherein R is benzyl, p-methoxybenzyl, allyl or propargyl, more preferably, benzyl.

6. The process to any one of the preceding claims, wherein the organoaluminiun halide is dimethyl aluminium chloride or diethylaluminium chloride.

7. The process according to any one of the preceding claims, wherein the azide reagent is selected from a metal salts of hydrazoic azid, a salt of hydrazoic acid with an organic base and an azide of formula R7R8MN₃ wherein M is boron or aluminium, preferably aluminium, R7 and R8 are, independently from one another, C₁₋₇alkyl, C₃-C₈alkenyl, C₃₋₈cycloalkyl, C₃₋₈cycloalkyl-C₁₋₇alkyl or C₆₋₁₀aryl-C₁₋₇alky, preferably C₁₋₇alkyl,

8. The process to any one of the preceding claims, wherein the azide reagent is of formula R7RBMN₃ wherein M is aluminium or boron, preferably aluminium, R7 and R8 are, independently from one another, C₁₋₇alkyl, C₃-C₈alkenyl, C₃₋₈cydoalkyl, C₃₋₈cycloalkyl-C₁₋₇alkyl or C₆₋₁₀aryl-C₁₋₇alkyl, preferably C₁₋₇alkyl, preferably, the azide reagent is diethylaluminium azide or dimethylaluminium azide, more preferably diethylaluminium azide.

9. The process according to claim 4, wherein the azide reagent is of formula R7R8MN₃, wherein M is aluminium, R7 and R8 are, independently from one another, C₁₋₇alkyl, C₃-C₈alkenyl, Ca₋gcycloalkyl, or C₆₋₁₀aryl-C₁₋₇alkyl, preferably C₁₋₇alkyl, and is formed in-situ by adding a metal salt of hydrazoic acid to the reaction mixture of step i), which an excess for example 2 or more equivalents, of the organoaluminiun halide reagent of formula as defined herein.

10. A of formula (III), or salt thereof, 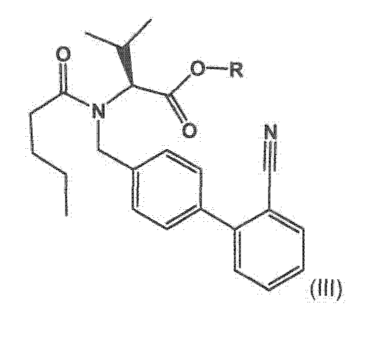 wherein R is benzyl, allyl, cinnamyl, or propargyl, more preferably, benzyl.

11. An amine salt of the of formula (IV)
